# EUROPEAN PATENT APPLICATION

(11) **EP 1 598 418 A1**
(43) Date of publication of application: **23.11.2005**
(21) Application number: 04703407.9
(22) Date of filing: 20.01.2004
(51) Int. Cl.: C12N 9/96

(54) **STABILISED COMPOSITION FOR FLUORIMETRIC, COLORIMETRIC OR CHEMOLUMINESCENT ASSAYS, KITS CONTAINING SAME AND PRODUCTION METHOD THEREOF**

(30) Priority: 26.02.2003 ES 200300472
(71) Applicant: BIOTOOLS BIOTECHNOLOGICAL & MEDICAL LABORATORIES, S.A., 28021 Madrid (ES)
(72) Inventor: MADEJON SEIZ, Antonio, E-28021 Madrid (ES); LIMONES LOPEZ, Gemma, E-28021 Madrid (ES); HARO CASTUERA, Amparo, E-28021 Madrid (ES); DE GRADO SANZ, Myriam, E-28021 Madrid (ES); FRANCO DE SARABIA ROSADO, Pedro Manuel, E-28021 Madrid (ES)
(74) Representative: Esteban Perez-Serrano, Maria Isabel
(86) International application number: PCT/ES2004/000024
(87) International publication number: WO 2004/076656

(57) **Abstract**

The invention relates to a stabilised composition containing: a component (A) which is selected from (i) a compound comprising a fluorophore, (ii) a compound comprising a first member of a specific binding pair which can recognise and interact with a second member of said specific binding pair, (iii) an enzymatic activity with catalyses a colorimetric or chemoluminescent reaction, (iv) a conjugate comprising an enzymatic activity which catalyses a colorimetric or chemoluminescent reaction and a member of a specific binding pair which can recognise and bind to a second member of said specific binding pair, (v) one or more compounds bound to a solid support, and mixtures thereof ; and a component (B) comprising a stabilising mixture. The invention can be used for fluorimetric, colorimetric or chemoluminiscent assays.

## Description

### FIELD OF THE INVENTION

The invention relates to a stabilised composition, useful for fluorimetric, colorimetric or chemo-luminescent assays, comprising the compounds and materials potentially used in said assays, such as molecules fixed to solid supports or otherwise, enzymatic activities, antibodies, proteins and fluorescent, colorimetric or chemoluminescent markers. The invention also relates to a procedure for obtaining said stabilised composition by the use of a stabilising mixture, as well as to the kits containing said composition.

### BACKGROUND OF THE INVENTION

The use of systems for detecting molecular markers based on fluorescence, colorimetry or chemoluminescence has become an alternative to the use of radioactive isotopes in recent years.

Colorimetry and chemoluminescence techniques are indirect detection systems based on the chemical modification of molecules with a high bonding capacity to specific target molecules, by the incorporation (conjugation) of enzymatic activities which, in the presence of the appropriate substrate, catalyse a reaction that generates a coloured compound (colorimetric reactions) or light (chemoluminescence). Said modified molecules are used as probes to identify the problem target molecules in complex mixtures. Finally, to determine the amount of conjugate bonded and indirectly the amount of target molecules present in the mixture, the target molecule / conjugate complexes are separated and the substrate of the chemical activity incorporated in the conjugate is added. The amount of product generated can be determined for colorimetric reactions by measuring the absorbance levels for specific wavelengths of the visible spectrum. For chemoluminescence reactions, the efficiency of the reaction is determined by measuring the light emitted with illuminometers or by imprinting photosensitive plates.

There exist a wide variety of enzymatic activities liable to catalyse colorimetric reactions that have been adapted to this type of assays. Among them can be noted as more commonly used peroxidase activity, which uses as a substrate several molecules such as 4-choloro-1-naphthol, 3-amino-4-ethylcarbazol, diaminobenzidyne, 3,3', 5, 5'-tetramethylbenzidyne (TMB), O-phenylendiamine (OPD), 2, 2'azinobis-(3-ethylbenzothiazoline-6-sulfonic acid) (ABTS) and 3-(p-hydroxyphenyl) (HPPA), among others. Also commonly used in colorimetry assays is alakaline phosphatase activity, using as substrates among others 3-(4-methoxyspiro[1,2-dioxethane-3'-2'-tricyclo-[3.3.1^{3,7}]decan]-4-yl)phenyl phosphate (AMPPD) and p-nitrophenyl phosphate (pNPP).

There also exist numerous luminescent markers among which are acridine esters, luminol, luciferine, etc. Also known are various enzymatic activities susceptible of generating chemo-luminescence reactions, such as the enzymatic activities: β-D-galactosidase, which uses adamantil-1.2-dioxyetane aryl galactoside (AMPGD) as a substrate, xantine oxidase on luminol + EDTA gold; glucose oxidase on luminol or isoluminol + microperoxidase; luciferase in the presence of luciferin + ATP; green fluorescent protein (GFP) in culture cells; etc. Similarly, isolated hot radish peroxidase (HRP) activity in the presence of luminol + perborate + 4-iodophenol or 4-hydroxicynamic acid; and alkaline phosphatase activity in the presence of AMPPD, 5-bromo-4-chloro-3-indolyl phosphate (BCIP), either in disodium or toluidine salt forms, or in the presence of nitrobluetetrazolium (NBT) salt, also generate chemoluminescent signals.

The enzymatic activity conjugation can be performed directly on a molecule, such as on an antigen or an antibody that respectively recognises the problem antibody or antigen. However, it is common to use intermediary molecules to bond the problem molecule to the conjugate. Among the most used intermediary molecules are those forming the systems avidin/biotin, streptavidin/biotin, avidin/phycoerythrin, digoxygenin/anti-digoxygenin, etc. Biotin (vitamin H, coenzyme R) is a small molecule that can bond to a great variety of proteins and nucleic acids without changing its properties. It also shows a highly specific bonding ability to avidin, a basically tetrameric glycoprotein abundant in egg white, and to streptoavidin, its equivalent synthesised by bacteria of the genus *Streptomyces.* The union of the avidin/biotin complex and avidin/phycoerythrin is highly stable, with a dissociation constant of 1.0 x 10⁻¹⁵ M and a dissociation energy of 21 kcal/mol. As avidin and streptoavidin can be easily complexed with various enzymatic activities involved in colorimetric or chemoluminescent reactions, the biotin/avidin or biotin/streptoavidin system is a system of choice in reactions for detecting and identifying nucleic acids, proteins (including antibodies), antigens, etc. Digoxygenin is a low molecular mass cardenolide that can be incorporated chemically as a ligand to DNA and RNA molecules without altering its capacity of hybridation to complementary nucleic acid sequences. Anti-digoxygenin antibodies have been designed in parallel that recognise this ligand specifically, to which a great variety of the aforementioned enzymatic activities can be complexed.

The biotin/avidin, biotin/streptoavidin and digoxygenin/antidigoxygenin systems have been widely used to develop techniques for detecting or identifying specific sequences of nucleic acids by colorimetry in a great variety of supports, including ELISA plate membranes and dimples (PCR ELISA) or chemoluminescence. This is because the chemical incorporation of biotin or digoxygenin molecules can be performed simply during the chemical synthesis of oligonucelotides, allowing to locate the marker at the desired position in the sequence without affecting the bonding ability of the oligonucleotide marked to complementary nucleic acid sequences. In addition, as the nucleotides marked with biotin or digoxygenin maintain their capacity to be used in nucleic acid elongation reactions catalysed by DNA polymerase activity, these systems are used to synthesise DNA or RNA probes used in subsequent hybridisation experiments.

An especially important case in the field of detection of biomolecules, particularly specific sequences of nucleic acids, is the use of fluorescent markers. Fluorescent materials are excited by radiation of a specific wavelength, returning to the state of equilibrium after emitting radiation at a wavelength different from the excitation wavelength. Fluorescence is mainly used in research laboratories in a great variety of experimental assays, such as detection of double-band nucleic acid molecules in agarose gels or polyacrylamide by identifying intercalated fluorescent molecules, such as ethidium bromide, etc. In more specific systems a fluorescent marker is introduced by chemical modification of protein or nucleic acid molecules. The modified molecules are then used as probes to identify target molecules recognised by the probe. This union can be direct or, as in the case of colorimetry and chemoluminescence reactions, by intermediary molecules.

The inclusion of fluorophores in proteins and polynucleotides is standardised. The case of oligonucleotides marked with fluorophores is of special interest as it is possible to include a great variety of fluorescent markers during the synthesis process.

Systems based on the use of fluorescent markers are especially useful for the simultaneous identification of several problem molecules in a single reaction mixture. Thus, unlike in colorimetric systems, the emission spectra of fluorescent compounds are well defined, covering a small range of wavelengths about the maximum emission wavelength. This has allowed to design an array of fluorescent compounds that emit different wavelengths and thus barely interfere with each other. Therefore, it is possible to include several different fluorescent markers in a single tube to obtain a separate identification without overlap of the problem molecules.

The ability to simultaneously discriminate different fluorescent markers in the same sample has allowed to develop gene sequencing systems using reactions of amplification or elongation of DNA sequences from specific nucleotides, in the presence of the four terminating nucleotides, each one marked with a different fluorophore. Finally, each fluorophore is discriminated in a single capillary electrophoresis array (capillary sequencing systems).

Possibly the field of greatest advances in recent years is real-time gene amplification reactions, which have allowed to add a quantitative dimension to the gene amplification reaction, the most sensitive technique for detecting nucleic acids. This technique consists of a simultaneous coupling of the gene amplification reaction and the detection reaction of the amplified material by fluorescent signals in each cycle of the amplification process. With this purpose, a growing number of units have been designed and commercialised that perform both function s simultaneously. The fluorescence reading incorporated in each amplification cycle allows to identify amplicons in the exponential amplification stage, where reaction saturation phenomena are not yet present. For this reason, the reflection of the initial amount of substrate DNA is much more accurate than in traditional methods, where the amplified product is analysed in later reaction phases.

The fluorescent marking that can be used in these techniques is diverse, comprising both the use of fluorescent intercalated substances in double-band DNA molecules (dsDNA), such as ethidium bromide, SYBR Green etc., and the use of primers and probes comprising a fluorophore and, optionally, a fluorescence modulation molecule (a quencher) such as Taqman probes, Molecular Beacon, Scorpion, FRET, etc.

Another application particularly used in recent years is the development of chips and arrays consisting of solid substrates of diverse nature (modified glasses, gold surfaces, modified plastic surfaces, etc.) on which nucleic acid or protein molecules are fixed, which are later used for specific detection of biomolecules in complex mixtures. The use of this type of supports allows two differentiated forms of use. This, a single molecule (such as nucleic acids or proteins) can be fixed on the support chip, which is then used to analyse a wide array of problem samples. This is the operating principle of some commercial chip systems, such as Biocore® chips (Izasa, Spain). However, other systems invert the process, having solid supports on which an array of molecules (nucleic acids or proteins) is previously fixed allowing to identify in a single assay a number of molecules of interest in complex biological samples. Depending on the number of different molecules of interest fixed on the chip support it is possible to find chips or arrays of low, medium and high density. In this sense, the development of robotic application systems allowing to handle volumes on the order of picolitres with high precision has allowed the development of microarrays of nucleic acids and proteins including thousands of different molecules. The use of such complex detection systems also requires to develop sophisticated result analysis systems. In this sense, fluorescent detection systems (microarrays) are commonly used, as well as electric conductivity analyses or light refraction angle modification (Biocore®) among others.

In view of the above, it can be seen that systems for marking biomolecules such as proteins and nucleic acids with fluorescent markers, or molecules involved in the development of colorimetric or chemoluminescent analysis techniques are currently common and essential techniques in laboratories performing diagnostic assays and biological research. However, to this day many of the techniques involved continue to require a great deal of handling by the researcher. Due to the great sensitivity of some of these systems, such as DNA or RNA quantification in real-time amplification systems, this handling can affect the reproducibility of the results.

For this reason, it would be important to have preformed and stabilised reaction systems that minimise reagent handling and limit the role of the handler to introducing the problem sample. This type of system would not only allow to increase the reproducibility of the results obtained in independent experiments, essential for their interpretation, but would also speed up the execution time of many different experimental techniques routinely used in the laboratory, thereby allowing to obtain the results faster.

Another important problem is the stabilisation of biomolecule chips or microarrays used as detection supports in complex biological samples. Such systems, particularly those having a large number of different molecules fixed, regardless of their nature (nucleic acids, proteins or other), require a correct stabilisation of all the molecules included in them to ensure a correct interpretation of the results of an experiment, as well as to ensure system reproducibility.

Systems for preparing and stabilising enzymatic activities have been developed. Patent application WO 93/00807 describes a system for stabilisation of biomaterials during the lyophilisation process. In addition, Biotools Biotechnological & Medical Laboratories S.A. have recently developed a system for gelling complex biomolecule mixtures that allows to stabilise reaction mixtures for long periods in diverse storage conditions (WO 02/07/2002). The use of this system has allowed to stabilise complex reaction mixtures such as mixtures for gene amplification reactions, which contain all the reagents needed to perform the experiment aliquoted in independent, ready-to-use phials in which it is only necessary to reconstitute the reaction mixture and add the problem nucleic acid. However, said patent application WO 02/07/2002 does not mention explicitly the addition of molecules marked with a fluorophore or those including enzymatic activities liable to catalyse colorimetric or chemoluminescent reactions, or intermediate molecules (ligands) in colorimetric or chemoluminescent detection systems. The inclusion of said molecules in a complex reaction system presents additional problems, such maintaining the bonding ability of the ligands to the receptors, maintaining enzymatic activities and, in the case of fluorescence reactions, non-interference with the emission or absorption spectra of the fluorescent markers included.

It has not been found that it is possible to stabilise reaction mixtures containing at least one compound involved in a fluorimetric, colorimetric or chemoluminescent assay selected among (i) a compound containing a fluorophore, (ii) a compound containing a ligand that can recognise and interact with a specific receptor, (iii) an enzymatic activity that catalyses a colorimetric or chemoluminescent reaction, (iv) a conjugate comprising an enzymatic activity that catalyses a colorimetric or chemoluminescent reaction, and a member of a specific binding pair with recognition and binding capacity to a second member of said specific binding pair, (v) one or more compounds, regardless of their nature, such as nucleic acids or proteins, joined to one or more solid supports, susceptible of being used in fluorimetric, colorimetric or chemoluminescent assays, or mixtures thereof, with a stabilising mixture comprising (i) at least one protective agent against desiccation; (ii) at least one inhibitor of the condensation reaction between the carbonyl or carboxyl group and the amine or phosphate group; and (iii) at least one inert polymer able to generate a grid structure that prevents the mobility of the desiccated reagents. Said reaction mixtures comprising at least one product involved in fluorimetric, colorimetric or chemoluminescent assays can optionally include at least one enzyme that catalyses the polymerisation of nucleic acids, as well as the components required to perform said polymerisation reaction.

### DETAILED DESCRIPTION OF THE INVENTION

### 1. Composition

In one of its aspects, the invention provides a stabilised composition, hereinafter the composition of the invention, comprising:
(i) a component (A) selected from the group formed by:
   - a compound comprising a fluorophore (component A1);
   - a compound comprising a first member of a specific binding pair able to recognise or interact with a second member of said specific binding pair (component A2);
   - an enzymatic activity that catalyses a colorimetric or chemoluminescent reaction (component A3);
   - a conjugate comprising an enzymatic activity that catalyses a colorimetric or chemoluminescent reaction and a member of a specific binding pair with capability of recognising and bonding to a second member of said specific binding pair (component A4);
   - one or more compounds joined to a solid support (component A5);
   - their mixtures; and
(ii) a component (B) constituted by a stabilising mixture that comprises:
   - at least one protective agent against desiccation (component B1);
   - at least one inhibitor of the condensation reaction between carbonyl or carboxyl groups and amino or phosphate groups (component B2); and
   - at least one inert polymer capable of generating a grid structure that prevents the mobility of the desiccated reagents (component B3).

The composition of the invention can optionally contain other components, such as water, enzymes, reagents for the reactions in which said enzymes participate, such as cofactors, substrates, additives to improve enzymatic reactions, etc.

In a specific embodiment, the composition of the invention comprises, in addition to components A and B, an enzyme that catalyses the polymerisation of nucleic acids, together with part or all of the components required to carry out said nucleic acid polymerisation reaction.

The composition of the invention can be obtained by desiccation in a vacuum without prior freezing. The composition of the invention has a humidity equal to or under 30%, preferably from 1% to 20%.

The composition of the invention can be used to perform fluorimetric, colorimetric or chemoluminescent assays in general, and more specifically to detect and/&or quantify macromolecular entities such as nucleic acids, proteins, antibodies, antigens, etc. by means of said fluorimetric, colorimetric or chemoluminescent assays. Advantageously, the composition of the invention is of the ready-to-use type.

In a specific embodiment, the composition of the invention is a stabilised gene sequencing system.

In a specific embodiment, the composition of the invention is embodied in a ready-to-use kit that includes all the components needed to perform a nucleic acid amplification reaction or gene sequencing reaction, including all the reagents to identify a predetermined marker, in the form of independent reaction phials so that the user only needs to add the problem sample in the appropriate amount and concentration.

In another specific embodiment, the composition of the invention comes in the form of a solid support (microplate, chip, array, microarray, etc.) with previously attached molecules (such as nucleic acids, proteins, etc.) stabilised by coating with the aforementioned stabilising mixture (B) and subsequent desiccation. Alternatively, in another embodiment the composition of the invention has the form of a solid support (microplate, chip, array, microarray, etc.) with attached molecules (such as nucleic acids, proteins, etc.) previously mixed with said stabilising mixture (B) and fixed by the specific methods used for each support.

### 1.1 Component A

a. The composition of the invention contains one or more components A1, A2, A3, A4 or A5 in the appropriate amounts to perform their function in the corresponding fluorimetric, colorimetric or chemoluminescent assay.

Component A1 (a compound containing a fluorophore) includes any compound containing a fluorescent molecular group or substance.

In a specific embodiment, component A1 is a fluorescent compound that can be introduced by a chemical interaction with formation of a true bond or by simple coupling in the molecule under study, without affecting its characteristics. Examples of such fluorescent compounds include acridins, such as orange acridin, proflavin, acriflavin, etc.; ethidium bromide; SYBR Green; fluorescein and its derivatives (such as carboxy, isothiocyanate, etc.); rhodamin B and its derivatives (such as isothiocyanate, etc.); 1-anilino-8-naphthalene sulfonate (ANS); dimethyl-amino-naphthalene-5-sulfonate (DNS) and its derivatives (such as chlorates, etc.); dansil chloride; 2-p-toluidinylnaphthalene-6-sulfonate (TNS); etc.

In another specific embodiment, the component A1 comprises a nucleotide, an oligonucleotide or a polynucleotide, regardless of their nature (DNA, RNA, DNA/RNA chimeras, etc.), optionally chemically modified, containing a fluorophore such as a nucleotide marked with a fluorescent compound, an oligonucleotide, as a primer, marked with a fluorescent compound, or a polynucleotide, such as a probe, marked with a fluorescent compound. Illustrative examples of component A1 according to this include deoxyribonucleotides triphosphate (dNTP's), such as dATP, dGTP, dCTP, dTTP, dUTP, marked with fluorescent compounds; oligonucleotides marked with fluorescent compounds on one of their ends, such as with LC Red 640 at end 5'; a FRET (Fluorescent Resonance Energy Transfer) probe system comprising a probe containing a donor fluorophore and another probe containing an acceptor fluorophore, which can hybridise with the amplification product or regions adjacent to the target DNA and emit a fluorescent signal that depends on the proximity of the fluorophores, such as a pair of oligonucleotide probes able to hybridise with adjacent regions in a DNA sample, one region marked with fluoroscein at the 3' terminating end and the other marked with LC Red 640 at the 5' terminating end; Taqman probes (WO 98/4046) comprising a probe marked with a fluorescent compound and a compound to modulate fluorescence (a quencher), such as a probe containing a FAM group (6-carboxy-fluoroscein) at the 5' terminating end and a TAMRA group (6-carboxy-tetramethyl-rhodamin) at the 3' terminating end; Molecular Beacon (MB) probes (WO 99/22018) comprising a probe marked with a fluorescent compound on one end and a quencher on the other, and a fork structure when it is not hybridised to a nucleic acid complementary of the nucleotide sequence of the loop, such as a MB probe containing fluorescein on the 5' terminating end and DABCYL on the 3' terminating end; Scorpion probes (US 2002/0102591) consisting on the union of a MB probe to a primer used in the amplification reaction .

In another specific embodiment, the component A1 comprises a fluorophore and a macromolecular entity other than a nucleic acid, for example a peptide, a protein, an antigen, etc., such as an antibody or antigen marked with a fluorescent compound, for example fluorescein and its derivatives, rhodamin B and its derivatives, ANS, DNS and its derivatives, TNS, dansyl chloride, etc.

Component A2 (a compound comprising a first member of a specific binding pair able to recognise and interact with a second member of said specific binding pair) relates to any compound containing a first member of a specific binding pair able to recognise and interact with a second member of said specific binding pair. Almost any specific binding pair can be used to create the composition of the invention. The composition of the invention will include at least one of the members of the specific binding pair. Examples of specific binding pairs are specific binding pairs useful to detect macromolecular entities such as nucleic acids, peptides, proteins, antigens, antibodies, compounds liable to be fixed by adsorption or by covalent bonding, etc., such as systems based on avidin or streptavidin, such as the specific binding pairs avidin (or streptavidin)/biotin, avidin/phycoerythrin, etc., as well as systems based on antigen/antibody interactions, or fragments thereof containing the recognition sites, such as the specific binding pair digoxygenin/antidigoxygenin, etc. By way of illustration, the component A2 eventually present in the composition of the invention can be the one used in many biomolecule detection systems in ELISA systems, this is, a primary antibody that recognises an antigen and can in turn be recognised by a secondary antibody bearing a fluorescent marker or conjugated with enzymatic activities. Other possible alternative systems can be an antigen or an antibody joined to biotin, an oligonucleotide joined to digoxygenin, etc.

Component A3 (enzymatic activity that catalyses a colorimetric or chemoluminescent reaction) relates to any enzymatic activity susceptible of catalysing a colorimetric or chemoluminescent reaction in the presence of the appropriate substrate, and therefore susceptible of being used in such reactions. Almost any enzymatic activity susceptible of catalysing a colorimetric or chemoluminescent reaction can be used to make the composition of the invention.

Examples of enzymatic activities susceptible of catalysing a colorimetric reaction include peroxidase activity, which among others uses as substrate 4-chloro-1-naphthol, 3-amino-4-ethylcarbazol, diaminobenzidyne, 3,3',5,5'-tetramethylbenzidyne (TMB), O-phenylendiamine (OPD), 2, 2'azinobis-(3-ethylbenzothiazoline-6-sulfonic acid) (ABTS) and 3-(p-hydroxyphenyl) (HPPA); the alkaline phospatase activity which among others uses as substrate 3-(4-methoxyspiro[1,2-dioxethane-3'-2'-tricyclo-[3.3.1^{3,7}]decan]-4-yl)phenyl phosphate (AMPPD) and p-nitrophenyl phosphate (pNPP).

Examples of enzymatic activities susceptible of catalysing chemo-luminescence reactions include the activity β-D-galactosidase, which uses adamantil-1.2-dioxyetane aryl galactoside (AMPGD) as a substrate, xantine oxidase activity on luminol + EDTA gold; glucose oxidase activity on luminol or isoluminol + microperoxidase; luciferase activity in the presence of luciferin + ATP; green fluorescent protein (GFP) in culture cells; isolated hot radish peroxidase (HRP) activity in the presence of luminol + perborate + 4-iodophenol or 4-hydroxicynamic acid; alkaline phospatase activity (AP) in the presence of AMPPD, 5-bromo-4-chloro-3-indolyl phosphate (BCIP), either in disodium or toluidine salt forms, or in the presence of nitrobluetetrazolium (NBT) salt; etc.

Component A4 (a conjugate comprising an enzymatic activity catalyses a colorimetric or chemoluminescent reaction and is a member of a specific binding pair able to recognise and bind to a second member of said specific binding pair) relates to a conjugate resulting from the union of an enzymatic activity susceptible of catalysing a colorimetric or chemoluminescent reaction enzymatic activity and a member of a specific binding pair able to recognise and bind to a second member of said specific binding pair. Said enzymatic activity that catalyses a colorimetric or chemoluminescent reaction can be any enzymatic activity susceptible of being used in said reactions, such any of those mentioned in relation to component A3. Similarly, the aforementioned member of a specific binding pair able to recognise and bind to a second member of said specific binding pair can be one of the members of the specific binding pairs mentioned in relation to component A2. By way of illustration, the component A4 eventually present in the composition of the invention can be a conjugate of the type avidin (or streptavidin)-enzymatic activity (HRP or AP) susceptible of catalysing a colorimetric or chemoluminescent reaction, such as the conjugate avidin (or streptavidin)-peroxidase, avidin (or streptavidin)- alkaline phospatase, an antibody able to bind to a molecule (antigen) that is conjugated with an enzymatic activity, such as HRP or AP, or a nucleic acid sequence conjugated with enzymatic activities (HRP or AP) which, by complementing bases and hybridisation, recognises other specific nucleic acid sequences.

Component A5 (one or more components joined to a solid component) relates to a solid support in which one or more compounds have been previously immobilised, susceptible of being used as a component of fluorimetric, colorimetric, chemoluminescent detection systems, electrical conductivity analysis systems, or index of refraction change systems, preferably in fluorimetric, colorimetric, chemoluminescent detection systems. The nature of the material constituting said solid support can vary greatly, and includes the necessary modifications to allow fixing the components, for example plastic (ELISA microplates), glasses (microarrays), gold surfaces (chips), etc. optionally treated to allow the adsorption or covalent bonding of macromolecules such as nucleic acids, proteins, etc. In a specific embodiment, said component A5 is an ELISA dimple microplate for both nucleic acids and proteins, in which one or more compounds of interest have been previously fixed (such as nucleic acids or proteins). In another specific embodiment, said component A5 comprises chips and microarrays with one or more compounds of interest immobilised on their surfaces, such as biomolecules (for example nucleic acids or proteins), regardless of the nature of the material constituting the chip or microarray, or of the modifications made in it required to allow fixing said compounds in the support.

### 1.2 Component B

The composition of the invention comprises a component B constituted of B1, B2 and B3. Component B will be present in the composition of the invention in the appropriate amount to allow performing its function in the corresponding reaction or assay.

Component B1 (desiccation protection agent) comprises at least a non-reducing carbohydrate with, optionally, a polyol. Preferably, said non-reducing carbohydrate is selected from the group formed by a non-reducing disaccharide, a non-reducing trisaccharide and their mixtures. Examples of non-reducing disaccharides that can be present in the composition of the invention include palatinitol (6-α-D-glucopyranosyl-manitol) and trehalose. Likewise, illustrative examples of non-reducing trisaccharides include rafinose and melezitose. The polyol is preferably selected from among glycerol, sorbitol and their mixtures; advantageously, the polyol is glycerol. Therefore, in a specific embodiment, the component B1 comprises a non-reducing carbohydrate selected from among palatinol, trehalose, rafinose, melezitose and their mixtures. In another specific embodiment, the component B1 comprises (i) a non-reducing carbohydrate selected from among palatinol, trehalose, rafinose, melezitose and their mixtures and (ii) a polyol selected from among glycerol, sorbitol and their mixtures.

Component B2 (an inhibitor of the condensation reactions between the carbonyl or carboxyl groups and amine or phosphate groups) inhibits the condensation reactions that may occur between the reactive carboxyl, carbonyl, amine and phosphate groups contained in the macromolecules present in the composition of the invention. Said inhibitor can be a competitive inhibitor or a non-competitive inhibitor. Among competitive inhibitors are amino acids, specifically α-aminoacids, such as natural α-aminoacids as lysine, arginine, tryptophane, etc., preferably lysine. Among non-competitive inhibitors, betaine, aminoguanidine and its derivatives have shown to be the most effective. The choice of the non-competitive inhibitor depends on the non-reducing carbohydrate used as a desiccation protection agent, so that in the presence of rafinose the most effective non-competitive inhibitor is betaine, while in the presence of other non-reducing inhibitors the most effective non-competitive inhibitors are aminoguanidine and its derivatives.

Component B3 (inert polymer able to form a grid structure that prevents the mobility of the desiccated reagents) improves the stability of the composition of the invention by generating a grid that prevents the mobility of the various components that compose said invention, so that they are immobilised to a greater or lesser extent in the cells formed by the polymer, thereby preventing said components from approaching each other to prevent any possible chemical reactions between the possible reactive groups. Component B3 must not react chemically with any of the components of the composition of the invention, and must create a sufficiently fine and malleable grid to allow trapping individual macromolecules without distorting their tertiary or quaternary structure. In a specific embodiment, component B3 is selected from the group formed by polyvinylpyrrolidone (PVP), polyethylene glycol (PEG) in various polymerisation degrees, dextrane, starch, Ficoll (a non-ionic polymer synthesised from saccharose), glucogen, Arabic gum and their mixtures. In general, glucogen and Arabic gum are the inert polymers that have shown a greatest effectiveness in their protective function. The amount of compound B3 present in the composition of the invention must be enough to ensure that a sufficiently dense grid is generated to prevent the mobility of the macromolecules, without later interfering after rehydration (reconstitution) of the composition of the invention nor in the reaction or the fluorescent, colorimetric or chemoluminescent assay to be performed.

### 1.3 Optional components

Optionally, the composition of the invention may contain one or more enzymes different from the enzymatic activities eventually present in the composition of the invention, along with all or part of the reagents needed to perform the reaction in which said enzymes take part, such as cofactors, substrates, additives improving enzymatic reactions, etc. Practically any enzyme may be present in the composition of the invention; however, in a specific embodiment said enzyme is an enzyme that catalyses the polymerisation of nucleic acids, such as nucleic acid amplification enzymes (whether thermostable or thermolabile), regardless of their chemical nature; in this case, the composition of the invention can also contain the components needed to perform said nucleic acid polymerisation reaction, such as reaction buffers, nucleotides, cofactors, etc.

### 1.4 Applications/uses of the composition of the invention

The composition of the invention has numerous applications, both in basic and applied research, such as in the analysis and study of all types of macromolecular entities (nucleic acids, peptides, proteins, antigens, antibodies, etc.). In fact, the composition of the invention is especially useful to perform fluorimetric, colorimetric or chemoluminescent assays. These assays can be used to detect, identify and/or quantify said macromolecular entities. In general, these assays can be performed easily using the composition of the invention, which will include the components needed for its intended use, after reconstitution such as by rehydration, and addition of the problem sample.

In a specific embodiment, the composition of the invention comprises a fluorophore intercalated between dsDNA's, or a compound comprising a fluorophore and, optionally, a quencher, said compound selected from among a nucleotide, an oligonucleotide and a polynucleotide, and in addition an enzyme that catalyses the polymerisation of nucleic acids. Said composition of the invention can be used to detect and/or quantify nucleic acids by a fluorimetric method. By way of example, said composition of the invention can be used:
- in the real-time amplification of nucleic acids, regardless of the system chosen (such as Taqman probes, MB probes, Scorpion probes, FRET probes, intercalated fluorophores, etc.) and the reaction format (such as conventional or capillary reaction tubes, plates, etc.);
- in nucleic acid sequencing reactions based on the use of fluorophores for identification, such as by PCR reactions using thermostable polymerases DNA or by nucleic acid elongation reactions in the presence of oligonucleotides or nucleotides marked with fluorophores; or
- in the identification of nucleic acid fragments in assays based on PCR's multiplexed with fluorescent compounds to identify fragments in capillary electrophoresis systems, etc.

In another specific embodiment, the composition of the invention comprises an oligonucleotide or polynucleotide chemically modified by including a compound that acts as a first member of a specific binding pair able to recognise or interact with a second member of said specific binding pair, in which the second member of the specific binding pair is linked to an enzymatic activity that catalyses a colorimetric or chemoluminescent reaction. Examples that illustrate this embodiment include oligonucleotides or polynucleotides joined to digoxygenin or biotin, which will act as the first binding pair, and which may interact with the second binding pair, such as an anti-digoxygenin antibody (for digoxygenin) or an anti-biotin antibody, with avidin or streptoavidin (for biotin). The second binding pair will be linked to an enzymatic activity that catalyses a colorimetric or chemoluminescent reaction. Said composition can be used to detect and/or quantify nucleic acids by a colorimetric or chemoluminescent method.

Detection and identification of nucleic acids is particularly interesting in diagnosis applications, such as to identify pathogens, or in prognosis applications, this is, to evaluate the risk of developing a specific pathology, for example detection of gene mutations associated to a higher risk of developing a pathology (cancer, Alzheimer's disease, etc.).

In another embodiment the composition of the invention comprises an antibody (that acts as a first binding pair) specifically bound to a second binding pair (an antigen). Said antibody can be conjugated with a fluorescent compound, such as fluorescein. Said composition can be used to detect and/or quantify antigens by an immuno-fluorescent method. Alternatively, the composition of the invention can contain said antibody conjugated with a first member of a binding pair, such as biotin, or a conjugate comprising an enzymatic activity that catalyses a colorimetric reaction, such as peroxidase, or a chemoluminescent reaction, such as β-D-galactosidase, and a second member of a specific binding pair (avidin or streptoavidin) able to recognise and bind to said first member (biotin) of said specific binding pair. This composition can be used to detect and/or quantify antigens by an immuno-colorimetric method or an immuno-chemoluminescent method. These compositions are useful to detect and identify antigens corresponding to tumours, viruses, etc. By way of example, this embodiment can be used to perform an ELISA test for detecting antigens, when used with either digoxygenin or biotin. The composition of the invention may or may not contain the conjugate in the same phial.

In another specific embodiment, the composition of the invention comprises an antigen susceptible of being specifically recognised by an antibody, conjugated with a fluorescent compound such as fluorescein. Said composition can be used to detect and/or quantify antigens by an immuno-fluorescent method. Alternatively, the composition of the invention can contain said antibody conjugated with a first member of a binding pair, such as biotin, or a conjugate comprising an enzymatic activity that catalyses a colorimetric reaction, such as peroxidase, or a chemoluminescent reaction, such as β-D-galactosidase, and a second member of a specific binding pair (avidin or streptoavidin) able to recognise and bind to said first member (biotin) of said specific binding pair. This composition can be used to detect and/or quantify antigens by an immuno-colorimetric method or an immuno-chemoluminescent method. These compositions are useful to detect and identify antigens corresponding to tumours, viruses, etc. By way of example, this embodiment can be used to perform an ELISA test for detecting antigens, when used with either digoxygenin or biotin. The composition of the invention may or may not contain the conjugate in the same phial, but will not include the reaction substrate.

In another specific embodiment the invention will consist of a solid support of any nature, such as ELISA microplates and microarray or chip supports, in which previously one or more molecular species of any nature have been immobilised. Said molecular species are stabilised by coating the support with the stabilisation mixture. In this case the format of the invention will be a support with linked and stabilised molecules. Said support can be used for colorimetric, fluorometric or chemoluminescent analysis, by any of the stabilised systems disclosed in this patent.

In another specific embodiment, the invention will consist of a stabilisation mixture that is added to the molecules prior to fixing them on a solid support, regardless of its nature, such as ELISA microplates and microarray or chip supports. The molecules thus stabilised will be applied on the solid support following the specific procedure for each support. In this embodiment, as in the previous case, the format of the invention will be a support with linked and stabilised molecules. Said support can be used for colorimetric, fluorometric or chemoluminescent analysis, by any of the stabilised systems disclosed in this patent.

### 2. Kits

In another aspect, the invention provides a kit comprising at least one container that contains a composition of the invention. The container may be any suitable container for the composition of the invention, such as a phial, a flask, etc.

In a specific embodiment, the kit provided by this invention is suitable for detecting and/or quantifying nucleic acids by colorimetric, fluorometric or chemoluminescent methods, and specifically for detecting by said methods nucleic acids amplified by real-time nucleic acid amplification, or in multiplexed PCR's, or resulting from sequencing reactions, etc. In another embodiment, the kit provided by this invention is suitable for detecting and/or quantifying peptides, proteins, antigens or antibodies by colorimetric, fluorometric or chemoluminescent methods.

Therefore, the kit of the invention will contain, in addition to the composition of the invention (with the appropriate components depending on its intended purpose), all or part of the components (reagents, factors, additives, etc.) needed to perform the reaction and related assay, except for the problem molecule (nucleic acid, peptide, protein, antigen, antibody, etc.).

By way of example, the invention provides a kit for real-time amplification and/or detection of specific sequences of nucleic acids (RNA or DNA) that at least comprises one container with a composition of the invention, stabilised and ready to use, said composition of the invention comprising an enzyme that catalyses the polymerisation of nucleic acids, such as a gene amplification or reverse transcription coupled to gene amplification enzyme, and in addition all the necessary reagents, including specific primers marked with fluorophores or probes comprising a fluorophore and, optionally, a quencher, such as Taqman, MB, FRET or Scorpion probes, except for the problem nucleic acid.

In another specific embodiment the invention provides a kit for real-time amplification and/or detection of specific sequences of nucleic acids (RNA or DNA) that at least comprises one container with a composition of the invention, stabilised and ready to use, said composition of the invention comprising an enzyme that catalyses the polymerisation of nucleic acids, such as a gene amplification or reverse transcription coupled to gene amplification enzyme, and in addition all the necessary reagents, including nucleotides marked with fluorescent markers, except for the problem nucleic acid.

In another specific embodiment the invention provides a kit for real-time amplification and/or detection of specific sequences of nucleic acids (RNA or DNA) that at least comprises one container with a composition of the invention, stabilised and ready to use, said composition of the invention comprising an enzyme that catalyses the polymerisation of nucleic acids, such as a gene amplification or reverse transcription coupled to gene amplification enzyme, and in addition all the necessary reagents, including fluorescent substances intercalated in dsDNA's, such as Sybr Green, except for the problem nucleic acid.

In another specific embodiment the invention provides a kit for sequencing nucleic acids by gene amplification that at least comprises one container with a composition of the invention, stabilised and ready to use, said composition of the invention comprising an enzyme that catalyses the polymerisation of nucleic acids, such as a gene amplification enzyme, and in addition all the necessary reagents, including fluorescent substances intercalated in dsDNA's, such as Sybr Green, except for the problem nucleic acid.

In another specific embodiment the invention provides a kit for sequencing nucleic acids by gene amplification that at least comprises one container with a composition of the invention, stabilised and ready to use, said composition of the invention comprising an enzyme that catalyses the polymerisation of nucleic acids, such as a gene amplification enzyme, and in addition all the necessary reagents, including primers or terminating nucleotides marked with a fluorescent marker.

In another specific embodiment the invention provides a kit for sequencing nucleic acids by nucleic acid elongation reactions that at least comprises one container with a composition of the invention, stabilised and ready to use, said composition of the invention comprising an enzyme that catalyses the polymerisation of nucleic acids, such as a gene amplification enzyme, and in addition all the necessary reagents, including primers or terminating nucleotides marked with a fluorescent marker.

In another specific embodiment the invention provides a kit for detecting nucleic acids that at least comprises one container with a composition of the invention, stabilised and ready to use, said composition of the invention comprising the necessary reagents, including compounds that contain a member of a specific binding pair conjugated with an enzymatic activity that catalyses a colorimetric or chemoluminescent reaction, such as a compound comprising an avidin or biotin group, or any other modification used for subsequent colorimetric or chemoluminescent analyses.

In another specific embodiment the invention provides a kit for detecting antibodies that at least comprises one container with a composition of the invention, stabilised and ready to use, said composition comprising a compound that contains a member of a specific binding pair conjugated with an enzymatic activity that catalyses a colorimetric or chemoluminescent reaction.

In another specific embodiment the invention provides a kit for detecting antigens that at least comprises one container with a composition of the invention, stabilised and ready to use, said composition comprising a compound that contains a member of a specific binding pair conjugated with an enzymatic activity that catalyses a colorimetric or chemoluminescent reaction.

Therefore, the present invention provides compositions (reaction mixtures) such as of the ready-to-use type and kits, closed and ready to use, that include (i) a compound containing a fluorophore; (ii) a compound comprising a ligand that can recognise and interact with a specific receptor, in which said ligand or receptor is linked to an enzymatic activity that catalyses a colorimetric or chemoluminescent reaction; (iii) an enzymatic activity that catalyses a colorimetric or chemoluminescent reaction; or (iv) a conjugate comprising an enzymatic activity that catalyses a colorimetric or chemoluminescent reaction and a member of a specific binding pair able to recognise and bind to a second member of said specific binding pair. Said compositions are useful for performing fluorescent, colorimetric or chemoluminescent assays. In general, when the composition of the invention comprise a member of a specific binding pair, the other member of said specific binding pair is contained in another container of the kit of the invention. In addition, kits intended for colorimetric or chemoluminescent assays will include a separate container with the substrate for said enzymatic activities that catalyse a colorimetric or chemoluminescent reaction.

The present invention simplifies the process of preparing amplification reactions using fluorescent, colorimetric or chemoluminescent markers by developing ready-to-use reaction systems in independent containers (phials). The development of ready-to-use reaction systems in independent containers allows a faster execution of many techniques commonly used in diagnosis and research laboratories, such as real-time amplification reactions, sequencing and marking with fluorescent groups or with intermediate groups in colorimetry and chemoluminescence reactions. In addition to speeding the execution of the experimental method, the reproducibility of the results is increased and the experimental error factor is greatly reduced.

In another specific embodiment the kit is a solid support, such as ELISA microplates, microarray supports or chips, containing immobilised and stabilised molecules that can be used in fluorimetric, colorimetric or chemoluminescence assays.

### 3. Procedure

### 3.1 Stabilisation of liquid mixtures

In another aspect, the invention provides a procedure for preparing a stabilised composition with a humidity level equal to or less than 30% (the composition of the invention), which involves:
a) bringing together in a single container:
   an aqueous solution that comprises at least a component (A) selected from the group formed by:
      - a compound comprising a fluorophore (component A1);
      - a compound comprising a first member of a specific binding pair able to recognise and interact with a second member of said specific binding pair (component A2);
      - an enzymatic activity that catalyses a colorimetric or chemoluminescent reaction (component A3);
      - a conjugate comprising an enzymatic activity that catalyses a colorimetric or chemoluminescent reaction and a member of a specific binding pair able to recognise and bind to a second member of said specific binding pair (component A4); and
      - the mixtures thereof; and
   an aqueous solution that comprises a component (B) consisting of:
      - at least one protection agent against desiccation (component B1);
      - at least one inhibitor of the condensation reaction between carbonyl or carboxyl and amino or phosphate groups (component B2); and
      - at least one inert polymer that can generate a grid structure that prevents the mobility of the desiccated reagents (component B3);
   to obtain an aqueous solution comprising said components A and B; and
b) removing all or part of the water contained in said aqueous solution that contains the components A and B obtained in stage a) until obtaining a composition that comprises said components A and B and has a humidity degree equal to or less than 30%.

The aqueous solution comprising component A can be prepared out of the container and added to it later as such, or it can be formed directly in the container by adding and mixing the various components of said aqueous solution in the container itself. Similarly, the aqueous solution comprising component B (stabilising mixture) can be prepared out of the container and added to it later as such, or it can be formed directly in the container by adding and mixing the various components in the container itself.

The container can be any appropriate vessel, such as a phial, made of any suitable material (glass, plastic, etc.).

Components A1-A4 and B1-B3 have been described previously in relation to the composition of the invention. The composition of the invention can also contain, if desired, one or more of the optional components mentioned before, such as one or more enzymes other than the enzymatic activities eventually present in the composition of the invention, such as enzymes (thermostable or thermolabile) for amplification of nucleic acids, restriction enzymes, enzymes involved nucleic acid amplification, sequencing or characterisation (identification) reactions etc., together with all or some of the reagents required to perform the reactions in which said enzymes participate, such as cofactors, substrates, additives that improve the enzymatic reactions, etc. Said optional components can be added to the aqueous solution containing component A or to the aqueous solution containing component B, or can be added to the aqueous solution resulting from mixing the aqueous solutions containing components A and B.

After mixing in the container the aqueous solution containing component A and the aqueous solution containing component B, an aqueous solution is obtained containing components A and B from which all or part of its water content is removed until a humidity level of 30% or less is reached, thereby obtaining a stabilised, fully or partially desiccated composition that comprises at least one component A and one component B that constitutes the composition of the invention.

The removal of all or part of the water present in the aqueous solution obtained by mixing the aqueous solution containing components A and B in the container can be performed by any conventional desiccation method, including for example lyophilisation, fluidised bed desiccation, desiccation at room temperature and atmospheric pressure, desiccation at room temperature and reduced pressure, desiccation at high temperature and atmospheric pressure, desiccation at high temperature and reduced pressure. The preferred desiccation method is desiccation at a temperature between 15°C and 60°C and a reduced pressure lower than atmospheric pressure. Other methods, such as those cited, may be applied for the desiccation but their greater cost, lower efficiency or greater aggression on the components of the reaction mixture to be desiccated discourage their use. In certain cases, such as when the reaction mixture is contained in a capillary tube, as the sample is deposited on the tube wall it may be necessary to centrifuge to make it reach the bottom.

In general, the desiccation level selected will mainly depend on economic factors (process cost, time required to reach a certain desiccation level, etc.) and on the ratio of the desiccation level to composition stability. For this reason, in a specific embodiment the remaining humidity level in the composition of the invention is from 1% to 20%. Completely desiccated compositions, i.e. those with a presence of residual water of 1 % or less, tend to have a lower stability (during storage) than those with a higher water percentage, with fully desiccated compositions showing a significant decrease in reaction yields after rehydration and addition of the reaction substrate. Although the composition of the invention, due to its stability, can be stored for one or more weeks at room temperature (25°C), it seems advisable that it be stored at temperatures from 4°C to 10°C to assure its correct operation in time.

During the desiccation process, component B1 stabilises the tertiary structure of the macromolecules present in the aqueous solution that comprises component A, replacing in this purpose the water molecules which, in an aqueous solution, form the protective cover that allows to maintain the three-dimensional structure of said macromolecules; it also blocks the reactions that may take place between the reactive chemical groups that may be present in said macromolecules, therefore exerting a stabilising effect on the desiccated compositions. Component B2 inhibits the condensation reactions that may occur between the carboxyl, carbonyl, amino and phosphate groups contained in the macromolecules present in the composition of the invention. Component B3 improves the stability of the composition of the invention by generating a grid that prevents the mobility of the various reagents composing it, so that they are immobilised to a greater or lesser extent in the cells formed by the polymer. Consequently, these reagents cannot approach one another and the chemical reaction of their surface reactive groups is prevented. The amount of inert polymer to be added must be enough to ensure that a sufficiently dense grid is generated to prevent the movement of the macromolecules, without interfering with subsequent reactions. The joint action of the three components (B1, B2 and B3) of the stabilising mixture (component B) allows the composition of the invention to be functional after storage. Addition of only one or two of said components B1, B2, B3 without the presence of the other component(s) will result in compositions that become inactive during the procedure of obtaining the composition of the invention or are unstable, their activity disappearing a few days after obtaining the composition of the invention, therefore having a very short stability in storage.

### 3.2 Stabilisation of molecules bound to solid supports

### 3.2.A Stabilisation of pre-immobilised molecules

In another aspect, the invention provides a procedure for preparing a stabilised compound that comprises a solid support with one or more immobilised and stabilised components, with a humidity equal to or under 30%, which comprises:

Bringing together a component (A), consisting of a solid support containing one or more compounds previously immobilised on said solid support, with a compound (B) consisting of a stabilising mixture that comprises:
- at least one protective agent against desiccation (component B1);
- at least one inhibitor of the condensation reaction between carbonyl or carboxyl groups and amine or phosphate groups (component B2); and
- at least one inert polymer able to generate a grid structure that prevents the mobility of the desiccated reagents (component B3); and

removing all or part of the water contained in the resulting mixture of said components (A) and (B) until obtaining a composition that comprises a solid support with one or more immobilised and stabilised components and said component B, with a humidity equal to or less than 30%.

Component (A) comprises a solid support made of plastic, glass or gold surfaces, optionally pre-treated to allow the adsorption or covalent bonding of the compounds. The compound or compounds immobilised in the solid support can be any compound of interest, for example one or more biomolecules such as nucleic acids (oligo- or poly- nucleotides, etc.) peptides, proteins, antibodies, etc. Said compounds are immobilised by conventional means on said solid support or, instead, the solid supports loaded with one or more compounds immobilised on them are purchased commercially. Component (B) is added to the solid support loaded with said compound(s) to form a thin layer coating said solid support, and finally all or part of the water present in the resulting mixture is removed by any conventional desiccation method, including for example lyophilisation, fluidised bed desiccation, desiccation at room temperature and atmospheric pressure, desiccation at room temperature and reduced pressure, desiccation at high temperature and atmospheric pressure, desiccation at high temperature and reduced pressure. The preferred desiccation method is desiccation at a temperature between 15°C and 60°C and a reduced pressure lower than atmospheric pressure. Other methods, such as those cited, may be applied for the desiccation but their greater cost, lower efficiency or greater aggression on the components of the reaction mixture to be desiccated discourage their use.

The stabilised composition that comprises a solid support containing one or more immobilised and stabilised compounds, with a humidity equal to or under 30%, that can be obtained according to the aforementioned process, can be used as a detection substrate in colorimetric, fluorescent or chemoluminescent systems or as a detection substrate in a method for analysis of results such as the electric conductivity or refractive index measurement. Therefore, the solid supports will fulfil the required conditions for use in such applications.

### 3.2B Fixing of stabilised molecules to solid supports

In another aspect, the invention provides a procedure for preparation of a stabilised composition that comprises a solid support containing one or more immobilised and stabilised compounds with a humidity level equal to or less than 30%, which involves:

Bringing together (i) a solid support, optionally treated to immobilise compounds, with (ii) one or more compounds stabilised by mixing said compound or compounds with a component (B), consisting of a stabilising mixture that comprises:
- at least one protective agent against desiccation (component B1);
- at least one inhibitor of the condensation reaction between carbonyl or carboxyl groups and amine or phosphate groups (component B2); and
- at least one inert polymer able to generate a grid structure that prevents the mobility of the desiccated reagents (component B3); and

removing all or part of the water contained in the resulting mixture until obtaining a composition that comprises a solid support with one or more immobilised and stabilised components and said component B, with a humidity equal to or less than 30%.

The solid support can be made of plastic, glass or gold surfaces, optionally pre-treated to allow the adsorption or covalent bonding of the compounds. The compound or compounds to be fixed in the solid support can be any compound of interest, for example one or more biomolecules such as nucleic acids (oligo- or poly- nucleotides, etc.), peptides, proteins, antibodies, etc. Said compounds are stabilised by placing them in contact with the aforementioned component (B), then adding them on the solid support optionally treated to immobilise said compounds. Then all or part of the water present in the resulting mixture is removed by any conventional desiccation method, including for example lyophilisation, fluidised bed desiccation, desiccation at room temperature and atmospheric pressure, desiccation at room temperature and reduced pressure, desiccation at high temperature and atmospheric pressure, desiccation at high temperature and reduced pressure. The preferred desiccation method is desiccation at a temperature between 15°C and 60°C and a reduced pressure lower than atmospheric pressure. Other methods, such as those cited, may be applied for the desiccation but their greater cost, lower efficiency or greater aggression on the components of the reaction mixture to be desiccated discourage their use.

The stabilised composition that comprises a solid support containing one or more immobilised and stabilised compounds, with a humidity equal to or under 30%, that can be obtained according to the aforementioned process, can be used as a detection substrate in colorimetric, fluorescent or chemoluminescent systems or as a detection substrate in a method for analysis of results such as the electric conductivity or refractive index measurement. Therefore, the solid supports will fulfil the required conditions for use in such applications.

The following examples illustrate the invention and should not be understood to limit its scope.

### EXAMPLE 1

### Preparation of a desiccated and stabilised reaction mixture with FRET (fluorescence resonance energy transfer) probes for real-time amplification

The thermostable DNA polymerase enzyme used in this and the following examples, unless otherwise stated, is a recombinant DNA polymerase of *Thermus thermophilus* expressed in *Escherichia coli,* property of Biotools B&M Labs S.A. España, and purified by a non-chromatographic method developed by the same company (BIOTOOLS DNA Polymerase). After purification, the enzyme was stored at -20°C in a storage buffer containing Tris HCl 30 mM, pH 8, glucose 25 mM, KCI 25 mM, PMSF 0.5 mM, Tween 20 0.25% and NP40 0.25%. A reaction buffer was prepared containing Tris HCl, pH 8, 750 mM, (NH₄)₂SO₄ 200 mM, Tween 20 0.1 % and MgCl₂ 20 mM.

Reaction mixtures were prepared for amplification of a fragment of approximately 750 base pairs (bp) corresponding to the coding region of fraction 18S of ribosomal RNA (rRNA from the small subunit, or SSUrRNA) of the genus *Plasmodium* (the amplified size presents small variations depending on the species). To do so, in amplification capillary tubes specific for use in the LightCycler System (Roche Applied Science, Mannheim Germany) were added 1 microlitre (1 U/µl) of DNA polymerase enzyme stored in its storage buffer, 2 microlitres of the reaction buffer and 1 microlitre of a solution containing equimolar proportions of the four deoxyribonucleotides (dNTP's) participating in the DNA amplification reaction (dATP, dCTP, dGTP and dTTP). In addition, 1µl in a concentration of 10mM was added of the primers A1 (^{5'}-AGT GTG TAT CAA TCG AGT TTC-^{3'}) and A2 (^{5'}-CGC AGT TGT TTG TCT CCA GAA-^{3'}), oligonucleotides whose purpose is to prime the amplification reaction. Finally, 1µl in a concentration of 10µM was added of the probes FRET A3 (^{5'}-TGT AAC TAT AGG GGA ACT F-^{3'}) and A4 (^{5'}-LCRed640-TTT AGC TTT TGG CTT TAA TAC-P-^{3'}). Both probes have the same polarity and hybridise in adjacent areas of the region amplified by the primers A1 and A2, leaving a separation of only two bases between the probes when hybridising. Probe A3 is marked with fluoresceine in locus 3' and probe A4 contains an LCRed640 group in end 5' and is phosphorylated in end 3' to prevent the elongation reaction primed by Taq polymerase, so that when the two probes hybridise the fluorescein and LC Red 640 groups are placed adjacent to each other, favouring the resonance process. Probes A3 and A4 were designed on a variable area of the *Plasmodium* genome, so that they hybridise with the amplified product of *P*. *falciparum,* but not with the amplified product of other *Plasmodium* species such as *P. vivax, P. malariae* or *P. ovale.*

Several tubes were prepared as described above, adding to each one suitable volumes of each of the stabilising mixtures shown in Table 1. The tubes thus prepared were centrifuged for 10 seconds at 4000 rpm in a tabletop centrifuge (Eppendorf) to ensure that the mixture remained at the bottom of the capillary tube. Finally they were desiccated in a centrifugal evaporator Eppendorf model 5301 at temperatures ranging from 10°C to 60°C for times ranging from 30 to 120 minutes. These temperatures and times will depend on the final volume of mixture to be desiccated.

After desiccation the tubes were conserved at the temperatures and for the times shown in Table 1. After the times shown in Table 1 had elapsed, their activity was tested by performing a gene amplification reaction on blood samples of patients infected with *P. falciparum, P. vivax, P. ovale* or *P*. *malariae.* To do so, 50 ng of DNA of each sample was added to each tube in a volume of 20 microlitres and the incubation cycles were performed, consisting of an initial incubation at 95°C for 10 minutes to allow hydration of the desiccated and stabilised mixture, followed by 45 cycles of denaturation (95°C, 10 seconds), annealing (55°C, 5 seconds) and extension (72°C, 10 seconds), with a temperature transition rate of 20°C per second in every case, using a LightCycler (Roche) thermocycler. In parallel, in order to check the evolution of the activity of the desiccated tubes, DNA samples were amplified using a fresh mixture in the same amplification conditions.

The result of the amplification reaction was analysed in every case by inspecting the fluorescence curve and determining the crossing point, following the instructions of the equipment software, for each reaction tube both fresh and desiccated and stabilised.

It was found in all cases that the stabilising mixtures containing melezitose or palatinitol in conjunction with lysine and glucogen or gum Arabic, or rafinose with betaine and glucogen, showed the greatest activity, with a variation coefficient at the crossing point of 2% with respect to the values obtained with the fresh samples. The samples of patients infected with *P*. *falciparum* gave positive results in every case, while patients infected with other species gave negative results in every case. On the contrary, when the amplified products were analysed in 1.5% agarose gel dyed with ethidium bromide, it was found that all samples included showed an amplification band of approximately 750 bp. These results confirmed that the amplification reaction had worked correctly in every case, and that the detection of *P*. *falciparum* was specific (Table 1).

The results obtained show the possibility of stabilising fluorescently marked probes without affecting their functionality. On another hand, the fact that the fluorescence curve has a growing behaviour during the process, without any fluorescent signal at the start of the reaction, suggests that the stabilising mixture used does not show a base fluorescence emission in the assay conditions.

### EXAMPLE 2

### Preparation of a reaction mixture desiccated and stabilised with TaqMan probes for real-time amplification

To test the efficiency of the TaqMan probes included in desiccated and stabilised reaction mixtures, reaction mixtures were prepared for amplification of the same region described for example 1, using the same reagents there described. To each capillary tube, specific for use in the Light Cycler System (Roche Applied Science, Mannheim, Germany) was added 1 microlitre of the aforementioned enzyme DNA polymerase (1 U/µl) conserved in its storage buffer, 2 microlitres of the aforementioned reaction buffer, 1 microlitre of a solution containing equimolar proportions of the four deoxyribonucleotides (dNTP's) participating in the DNA amplification reaction (dATP, dCTP, dGTP and dTTP). In addition, 1 µl in a concentration of 10mM was added of the primers A1 (^{5'} -AGT GTG TAT CAA TCG AGT TTC-^{3'}) and 1 microlitre of primer A2 (^{5'}-CGC AGT TGT TTG TCT CCA GAA-^{3'}) at a concentration of 10µM. Also to each phial was added 1 microlitre of the hydrolysis probe A5 (TaqMan probe: ^{5'}-FAM TTT AGC TTT TGG CTT TAAFTAC-TAMRA-^{3'}) at a concentration of 10mM, containing a FAM group at end 5'and a TAMRA group at locus 3'. The hybrid A5 probe hybridises with the genome of *P. falciparum* but does not recognise the amplified product of other *Plasmodium* species as *P. vivax, P. malariae* or *P. ovale.* Various tubes were prepared as described above, adding to each the suitable volume of each stabilising mixture shown in Table 1. The tubes thus prepared were centrifuged for 10 seconds at 4000 rpm in a tabletop centrifuge (Eppendorf). Finally, they were desiccated in a centrifugal evaporator Eppendorf model 5301 at temperatures ranging from 10°C to 60°C for times ranging from 30 to 120 minutes. These temperatures and times will depend on the final volume of mixture to be desiccated.

After desiccation the tubes were conserved at the temperatures and for the times shown in Table 1. After the times shown in Table 1 had elapsed, their activity was tested by performing a gene amplification reaction on blood samples of patients infected with *P. falciparum, P. vivax, P. ovale* or *P. malariae.*

To do so, 50 ng of DNA of each problem sample was added to each tube to a final volume of 20 microlitres. Then the incubation cycles were performed, which consisted of an initial incubation at 95°C for 10 minutes to allow hydration of the desiccated and stabilised mixture, followed by 45 cycles of denaturation (95°C, 10 seconds), annealing (55°C, 5 seconds) and extension (72°C, 10 seconds), with a temperature transition rate of 20°C per second in every case, using a LightCycler (Roche) thermocycler. In parallel, in order to check the evolution of the activity of the desiccated tubes, DNA samples were amplified using a fresh mixture in the same amplification conditions.

The result of the amplification reaction was analysed in every case by inspecting the fluorescence curve and determining the crossing point, following the instructions of the equipment software, for each reaction tube both fresh and desiccated and stabilised. It was found in all cases that the stabilising mixtures containing melezitose or palatinitol in conjunction with lysine and glucogen or gum Arabic, or rafinose with betaine and glucogen, showed the greatest activity, with a variation coefficient at the crossing point of 2% with respect to the values obtained with the fresh samples. The samples of patients infected with *P. falciparum* gave positive results in every case, while patients infected with other species gave negative results in every case. On the contrary, when the amplified products were analysed in 1.5% agarose gel dyed with ethidium bromide, it was found that all samples included showed an amplification band of approximately 750 bp. These results confirmed that the amplification reaction had worked correctly in every case, and that the detection of *P. falciparum* was specific (Table 1).

### EXAMPLE 3

### Preparation of a desiccated and stabilised reaction mixture with a Molecular Beacon (MB) probe system for real-time amplification

To test the efficiency of MB probes included in desiccated and stabilised reaction mixtures, reaction mixtures were prepared for amplifying the same region described in Example 1, using the same reagents as described. In this case the unit "DNA Engine OPTICON™ 2 System" (MJ Research) was used. In 0.2 ml amplification tubes with an optical lid, specific for use in the OPTICON system, were added 1 microlitre of DNA polymerase (1 U/µl) conserved in its storage buffer, 2.5 microlitres of the reaction buffer, 1 microlitre of a solution containing equimolar proportions of the four deoxyribonucleotides (dNTP's) participating in the DNA amplification reaction (dATP, dCTP, dGTP and dTTP), 1 microlitre of primer A1 (^{5'}-AGT GTG TAT CAA TCG AGT TTC-^{3'}) and 1 microlitre of primer A2 (^{5'}-CGC AGT TGT TTG TCT CCA GAA-^{3'}), both at a concentration of 10µM. Also to each phial was added 1 microlitre of the MB probe A6 (^{5'}-F-**CCT GC**T GTA ACT ATT CTA GGG GAA CTG CAG G-DABCYL-^{3'}) in a concentration of 10µM, containing a fluorescein group (F in the sequence) at end 5'and a DABCYL group at end 3' acting as a quencher. The first 5 nucleotides are complementary of the last 5 (shown in boldface in the sequence), allowing the oligo to acquire a fork-like configuration that allows the spatial approximation of the fluorescein group to the quencher, thereby inhibiting the fluorescence emission. In the presence of the specific sequence amplified by the other two oligonucleotides (A1 and A2) present in the reaction mixture, the central region of 21 bp of the oligonucleotide A6 hybridises with the amplicons, thereby breaking the fork structure and allowing the fluorescence emission by the fluorescein group, as it is spatially distanced from the DABCYL group. The central region of probe A6 hybridises with the genome of *P*. *falciparum* but does not recognise the amplified product of other *Plasmodium* species as *P. vivax, P. malariae* or *P. ovale.*

Various tubes were prepared as described above, adding to each the suitable volume of each stabilising mixture shown in Table 1. Finally, they were desiccated in a centrifugal evaporator Eppendorf model 5301 at temperatures ranging from 10°C to 60°C for times ranging from 30 to 120 minutes. These temperatures and times will depend on the final volume of mixture to be desiccated.

After desiccation the tubes were conserved at the temperatures and for the times shown in Table 1. After the times shown in Table 1 had elapsed, their activity was tested by performing a gene amplification reaction on blood samples of patients infected with *P. falciparum, P. vivax, P. ovale* or *P*. *malariae.*

To do so, 50 ng of DNA of each problem sample was added to each tube to a final volume of 25 microlitres. Then the incubation cycles were performed, which consisted of an initial incubation at 95°C for 10 minutes to ensure the rehydration of the desiccated and stabilised mixture, followed by 45 cycles of denaturation (95°C, 30 seconds), annealing (55°C, 30 seconds) and extension (72°C, 30 seconds) using a "DNA Engine OPTICON™ 2 System". In parallel, in order to check the evolution of the activity of the desiccated tubes, DNA samples were amplified using a fresh mixture in the same amplification conditions.

The result of the amplification reaction was analysed in every case by inspecting the fluorescence curve and determining the crossing point, following the instructions of the equipment software, for each reaction tube both fresh and desiccated and stabilised. It was found in all cases that the stabilising mixtures containing melezitose or palatinitol in conjunction with lysine and glucogen or gum Arabic, or rafinose with betaine and glucogen, showed the greatest activity, with a variation coefficient at the crossing point of 2% with respect to the values obtained with the fresh samples. The samples of patients infected with *P*. *falciparum* gave positive results in every case, while patients infected with other species gave negative results in every case. On the contrary, when the amplified products were analysed in 1.5% agarose gel dyed with ethidium bromide, it was found that all samples included showed an amplification band of approximately 750 bp. These results confirmed that the amplification reaction had worked correctly in every case, and that the detection of *P. falciparum* was specific (Table 1).

### EXAMPLE 4

### Preparation of a reaction mixture with Scorpion probes for real-time amplification

To test the efficiency of Scorpion probes included in desiccated and stabilised reaction mixtures, reaction mixtures were prepared for amplifying a 125 bp region of the rpo gene of *mycobacterium tuberculosis,* using a Light Cycler unit and the same reagents described in example 1. To each capillary reaction tube, specific for use in the Light Cycler system (Roche Applied Science, Mannheim, Germany) was added 1 microlitre of enzyme DNA polymerase (1 U/µl) conserved in its storage buffer, 2 microlitres of the reaction buffer, 1 microlitre of a solution containing equimolar proportions of the four deoxyribonucleotides (dNTP's) participating in the DNA amplification reaction (dATP, dCTP, dGTP and dTTP) and 1 microlitre of primer A7 (^{5'}-GAC AGC CAG CCG ATC AGA CCG-^{3'}) at a concentration of 10µM. Also to each phial was added 1 microlitre of the feeder A8 (Scorpion probe: ^{5'}-FAM-CCGCGACGCACCTCCAGCCCGGCACGCTGGCGCT MR HEG CCCGGCGGTCTGTCACGTG-^{3'}) in a concentration of 10mM, containing a FAM group in locus 5', a methylred (MR) joined to locus 1 of the deoxyribose of a central nucleotide and a hexylethyleneglycol group (HEG) acting as an inhibitor of the elongation.

Various tubes were prepared as described above, adding to each the suitable volume of each stabilising mixture shown in Table 1. They were then centrifuged for 10 seconds at 4000 rpm in a tabletop centrifuge (Eppendorf). Finally, they were desiccated in a centrifugal evaporator Eppendorf model 5301 at temperatures ranging from 10°C to 60°C for times ranging from 30 to 120 minutes. These temperatures and times will depend on the final volume of mixture to be desiccated.

After desiccation the tubes were conserved at the temperatures and for the times shown in Table 1. After the times shown in Table 1 had elapsed, their activity was tested by performing a gene amplification reaction on blood samples of patients infected with *M. tuberculosis* and samples of non-infected patients.

To do so, 50 ng of DNA of problem DNA was added to each tube to a final volume of 20 microlitres. Then the incubation cycles were performed, which consisted of an initial incubation at 95°C for 10 minutes followed by 45 cycles of denaturation (95°C, 10 seconds), annealing (55°C, 5 seconds) and extension (72°C, 10 seconds) with a temperature transition rate of 20°C per second in every case, using a LightCycler (Roche) thermocycler. In parallel, in order to check the evolution of the activity of the desiccated and stabilised reaction mixtures, DNA samples were amplified using a fresh mixture in the same amplification conditions.

The result of the amplification reaction was analysed in every case by inspecting the fluorescence curve and determining the crossing point, following the instructions of the equipment software, for each reaction tube both fresh and desiccated and stabilised. It was found in all cases that the stabilising mixtures containing melezitose or palatinitol in conjunction with lysine and glucogen or gum Arabic, or rafinose with betaine and glucogen, showed the greatest activity, with a variation coefficient at the crossing point of 2% with respect to the values obtained with the fresh samples (Table 1). The samples of patients infected with *M. tuberculosis* gave positive results in every case, while samples obtained from healthy donors gave negative results in every case.

### EXAMPLE 5

### Preparation of a desiccated and stabilised reaction mixture with SYBR Green for real-time amplification

To test the efficiency of intercalated fluorophores such as SYBR Green included in desiccated and stabilised reaction mixtures, reaction mixtures were prepared for amplifying a 250 bp fragment corresponding to region E6-E7 of oncogenic papillomaviruses, using the same reagents as described in Example 1. In this case the unit "DNA Engine OPTICON™ 2 System" (MJ Research) was used. In 0.2 ml amplification tubes with an optical lid, specific for use in the OPTICON system, were added 1 microlitre of DNA polymerase (1 U/µl) conserved in its storage buffer, 2.5 microlitres of the reaction buffer, 1 microlitre of a solution containing equimolar proportions of the four deoxyribonucleotides (dNTP's) participating in the DNA amplification reaction (dATP, dCTP, dGTP and dTTP), 1 microlitre of primer A9 (^{5'}-TGTCAAAAACCGTTGTGTCC-^{3'}) and 1 microlitre of primer A10 (^{5'}-GAGCTGTCGCTTAATTGCTC-^{3'}), both at a concentration of 10µM. Also to each phial was added 0.3 MI of SYBR Green 1 (Molecular probes).

Various tubes were prepared as described above, adding to each the suitable volume of each stabilising mixture shown in Table 1. Finally, they were desiccated in a centrifugal evaporator Eppendorf model 5301 at temperatures ranging from 10°C to 60°C for times ranging from 30 to 120 minutes. These temperatures and times will depend on the final volume of mixture to be desiccated.

After desiccation the tubes were conserved at the temperatures and for the times shown in Table 1. After the times shown in Table 1 had elapsed, their activity was tested by performing a gene amplification reaction on blood samples of patients infected with oncogenic or non-oncogenic papillomaviruses.

Then the incubation cycles were performed, which consisted of an initial incubation at 95°C for 10 minutes to ensure the rehydration of the desiccated and stabilised mixture, followed by 45 cycles of denaturation (95°C, 30 seconds), annealing (55°C, 30 seconds) and extension (72°C, 30 seconds) using a "DNA Engine OPTICON™ 2 System". In parallel, in order to check the evolution of the activity of the desiccated tubes, DNA samples were amplified using a fresh mixture in the same amplification conditions.

The result of the amplification reaction was analysed in every case by inspecting the fluorescence curve and determining the crossing point, following the instructions of the equipment software, for each reaction tube both fresh and desiccated and stabilised. It was found in all cases that the stabilising mixtures containing melezitose or palatinitol in conjunction with lysine and glucogen or gum Arabic, or rafinose with betaine and glucogen, showed the greatest activity, with a variation coefficient at the crossing point of 2% with respect to the values obtained with the fresh samples. The samples of patients infected with oncogenic HPV gave positive results in every case, while patients infected with non-oncogenic HPV, or not infected with HPV, gave negative results in every case. On the contrary, when the amplified products were analysed in 1.5% agarose gel dyed with ethidium bromide, it was found that all samples included showed an amplification band of approximately 250 bp. These results confirmed that the amplification reaction had worked correctly in every case, and that the detection of oncogenic HPV species was specific (Table 1).

A particularly striking fact was the reproducibility level found when performing the amplification reactions with the desiccated and stabilised reaction mixtures, encountering that the fluorescence profiles during the amplification process were practically superimposed on each other in two tested samples, while reactions performed with a fresh mixture of the same composition showed a greater variability. In addition, although the graphs of the desiccated and stabilised mixture reagents had a flatter profile, particularly in the final stage of the amplification process, the crossing point data were practically identical as those obtained with the fresh samples.

Finally, the maintenance of activity in the desiccated and stabilised reaction mixtures including activity Taq polymerase and intercalated SYBR Green for a period of up to one month ensures that, in the stabilisation conditions used, the coexistence of these molecules does not imply a loss in activity of Taq polymerase.

### EXAMPLE 6

### Preparation of desiccated and stabilised reaction mixtures using fluorescent markers

To test the possibility of gelling reaction mixtures for sequencing nucleic acids the CEQ 200 Dye Terminator Cycle Sequencing (DTCS) Quick Start Kit (Beckman Coulter) was employed, which uses a PCR sequencing system in a single tube by incorporating four different fluorescent terminators. The sequencing reactions were later analysed in a CEQ 2000 XL DNA Analysis System (Beckman Coulter) capillary electrophoresis automatic sequencing system.

Reaction mixtures were prepared containing 4 microlitres of DTCS Quick Start Master Mix, 1 microlitre of universal primer M13-17 and the suitable volume of each stabilising mixture shown in Table 1. The tubes thus prepared were desiccated in a centrifugal evaporator Eppendorf model 5301 at temperatures ranging from 10°C to 60°C for times ranging from 30 to 120 minutes. These temperatures and times will depend on the final volume of mixture to be desiccated.

After desiccation the tubes were conserved at the temperatures and for the times shown in Table 1. After the times shown in Table 1 had elapsed, their activity was tested by performing a gene amplification reaction on a pONC plasmid containing a cloned 250 bp fragment of the genome of human papillomavirus in the poly-linker of plasmid pBluescript II SK (-) following the instructions of the sequencing kit.

The incubation cycles were then performed, which consisted of an initial incubation at 95°C for 10 minutes, followed by 30 cycles of denaturation (95°C, 20 seconds), annealing (55°C, 20 seconds) and extension (60°C, 4 minutes) using a Minicycler (MJ Research) thermocycler. In parallel, in order to check the evolution of the activity of the desiccated and stabilised tubes, the same plasmid was sequenced using a fresh sequencing mixture in standard conditions. Finally, the sequencing reactions were analysed in the CEQ 200XL system following the supplier's instructions.

The data obtained show that stabilising mixtures containing melezitose or palatinitol in conjunction with lysine and glucogen or gum Arabic, or rafinose with betaine and glucogen, were efficient in the sequencing reactions. A comparison of the sequences obtained with the desiccated and stabilised mixtures and the standard sequencing mixtures showed that the reaction yields were very similar. Thus, the number of bases read with the desiccated and stabilised system (638) was similar to that obtained with the standard system (686). The percentage of coincidence with the theoretical sequence was analysed in the 593 coinciding bases in both readings, finding that the behaviour of the desiccated and stabilised mixture and the standard mixture was similar. Thus, the homology percentage of the sequence obtained with the desiccated and stabilised mixture with respect to the theoretical sequence was 572/593 (96.5%) of the bases analysed and 577/593 (97%) for the standard sequence.

These results confirm that the stabilisation of reaction mixtures simultaneously with four fluorophores is possible without affecting the functionality of the system.

### EXAMPLE 7

### Preparation of amplification reactions with avidin/biotin and subsequent colorimetric assay

### 7.A Use of oligonucleotides chemically modified and stabilised by desiccation in gene amplification reactions

The possibility was studied of stabilising reaction mixtures containing oligonucleotides chemically modified by including biotin and/or digoxygenin groups, and their functionality as primers in gene amplification reactions, as well as the persistence of their ability to bind to ligands (streptoavidin and anti-digoxygenin antibodies respectively), commonly used in colorimetry assays. To do so, 4 oligonucleotides were designed that hybridised with the genome of region E6-E7 of oncogenic HPV species. Two of the oligonucleotides, A9 (^{5'}-TGTCAAAAACCGTTGTGTCC-^{3'}) and A11 (^{5'}-Bio-TGTCAAAAACCGTTGTGTCC-^{3'}), with positive polarity, had identical sequences except in that one (A11) included a biotin group (Bio in the sequence) in locus 5'. The other two nucleotides, A10 (^{5'}-GAGCTGTCGCTTAATTGCTC-^{3'}) and A12 (^{5'}-Dig-GAGCTGTCGCTTAATTGCTC-^{3'}) had inverse polarity, and as in the previous case their nucleotide sequence was identical except in that one (A12) included a digoxygenin group (Dig in the sequence) in locus 5'.

Four different stabilising mixtures were prepared as described: In 0.2 ml amplification tubes were added 1 microlitre of polymerase DNA (1U/µl) conserved in its storage buffer, 5 microlitres of the reaction buffer and 1 microlitre of a solution containing equimolar proportions of the four deoxyribonucleotides (dNTP's) participating in the DNA amplification reaction (dATP, dCTP, dGTP and dTTP). Then 1 microlitre of each primer was added in a concentration of 20µM as follows:
Stabilising mixture 1: Unmodified primer (A9) + Primer with digoxygenin (A12)
Stabilising mixture 2: Unmodified primer (A10) + Primer with biotin (A11)
Stabilising mixture 3: Primer with digoxygenin (A12) + Primer with biotin (A11)
Stabilising mixture 4: Unmodified primers (A9 + A10).

### 7.B Maintenance of the binding capacity of oligonucleotides chemically modified and stabilised by desiccation in the presence of a stabilising mixtures to their ligands

To test the maintenance of the binding capacity of the biotin and digoxygenin groups stabilised by desiccation in the presence of a stabilising mixture to the ligands commonly used in colorimetry tests (streptoavidin and antidigoxygenin antibodies, respectively), an ELISA plate assay was performed using the amplified products obtained in Example 7.A.

To do so, an ELISA microplate with a high-binding capacity to streptoavidin (Biospa) was activated following a standard fixing protocol. Then, after blocking the plate by incubation with gelatine, the amplified products of the stabilising mixtures 1, 2 and 3 described in Example 7.A were analysed by a colorimetry assay according to the following experimental process:
A.- Amplification products using an onligonucleotide marked with digoxygenin (mixture 1 in Example 7.A). The biotinated probe A11 was fixed to the plate and the amplified product was hybridised. Then the hybrid was incubated with antidigoxygenin conjugated with alkaline phospatase antibody (Sigma) and it was revealed colorimetrically using pNpp (Roche) as substrate, analysing the absorbance levels at 415 nm with a plate reader (Beckman).
B.- Products amplified using primers marked with biotin (mixture 2 in Example 7.A). The amplification product was fixed to the plate with the first biotinilate and then hybridised with the first A12 marked with digoxygenin. The results were analysed as described in the prior step.
C.- Products amplified simultaneously with primers marked with biotin and digoxygenin (mixture 3 in Example 7.A). The amplification product was fixed to the plate with the first biotinilate and incubated directly with the conjugate antidigoxygenin/AP, revealing the result as described in sections A and B of this experiment.

In all cases the plate blocking, hybridising, washing and colorimetric revealing reactions were performed according to the protocol recommended by the DNA-Bind plate system from Costar.

The results of the colorimetric assay showed that both the biotin and digoxygenin groups in the desiccated and stabilised reaction mixtures maintain their binding capacity to streptoavidin and antidigoxygenin antibodies respectively. Thus, all the products amplified with primers modified with biotin and digoxygenin simultaneously yielded an intense colorimetry signal, showing the binding capacity of the biotin group to the streptoavidin fixed in the plate, as well as the union of digoxygenin to its conjugated antibody. These results were confirmed by the appearance of a colorimetric signal in the products amplified with biotin or digoxygenin, which produced an intense signal when hybridising with their specific probe. No unspecific signals were observed in the inactivated dimples nor when hybridising with primers not specific to the amplified product.

Finally, when the colorimetric analysis assay was repeated on amplifications of seried dilutions of the plasmid plnc, using the stabilising mixture 1 described in Example 7.A (amplification with an oligonucleotide marked with digoxygenin) and analysed as described in this section, it was confirmed that the use of primers marked with biotin or digoxygenin and stabilised by desiccation fully maintain their amplification and binding to ligand capacity, allowing their use in colorimetric assays, obtaining a good level of reproducibility in different experiments (Table 1).

### EXAMPLE 8

### Stabilisation of antibodies and proteic activities involved in colorimetric assays

The possibility was studied of stabilising various conjugates such as streptoavidin conjugated with HRP (Str-HRP) and anti-digoxygenin conjugated with alkaline phospatase (anti-DIG-AP) involved in colorimetric assays.

In 0.2 ml amplification tubes were added 100 microlitres of a 1/250 dilution in a blocking solution of the conjugate Str-HRP (Biospa). In parallel, 0.2 ml phials were prepared in which 100 microlitres were added of a 1/250 dilution in a blocking solution of anti-DIG-AP (Sigma). The suitable volume of stabilising mixture was added to each phial as shown in Table 1. The tubes thus prepared were desiccated in a centrifugal evaporator Eppendorf model 5301 at temperatures ranging from 10°C to 60°C for times ranging from 30 to 120 minutes. These temperatures and times will depend on the final volume of mixture to be desiccated.

After desiccation, the tubes were conserved at the temperatures and for the time shown in Table 1. After the times shown in Table 1 had elapsed, the desiccated and stabilised tubes were resuspended in 100 microlitres of sterile distilled H₂O and their activity was tested by performing colorimetric detection test in an ELISA plate of amplified products of oncogenic HPV (HPV 16), obtained by amplifying the plasmid pOnc using primers marked with digoxygenin (A12) and biotin (A11), using a desiccated and stabilised reaction mixture as described in Example 7.A, stabilising mixture 3.

For the activity assay of the conjugate anti-DIG-AP an ELISA plate was used in which the dimples had been previously coated with streptoavidin. For the activity assay of conjugate Str-HRP a plate was used instead with dimples previously coated with anti-DIG antibodies. The amplified products were incubated in each plate to allow the immobilisation of the amplicons in the plate. Finally, the conjugate to be tested was added to each plate and revealed, using as reaction substrate pNpp in the case of AP activity or for HRP activity. Finally, the results were analysed in a plate reader, measuring absorbance at 405 nm.

The results obtained were positive for all desiccated and stabilised mixtures, as well as similar to those obtained when both conjugates were used in a standard, non-desiccated format. These data confirm that streptoavidin and anti-DIG antibodies maintained their binding capacity to their specific ligands (biotin and digoxygenin respectively). On another hand, they confirm the maintenance of the enzymatic activity of the conjugated peroxidase and alkaline phosphatase (Table 1).

### EXAMPLE 9

### Stabilisation of reaction mixtures for synthesis of marked probes for their later use in molecular hybridisation experiments

The possibility was analysed of synthesising probes marked with biotin or digoxygenin groups for subsequent testing in molecular hybridisation experiments. To do so, 0.2 ml tubes were prepared in which were added 1 microlitre (1 U/µl) of DNA polymerase stored in its storage buffer, 5 microlitres of the reaction buffer, 1 microlitre of a solution containing equimolar proportions of the four deoxyribonucleotides (dNTP's) participating in the DNA amplification reaction (dATP, dCTP, dGTP and dTTP), 1µl of primer A10 in a concentration of 20µM and primer A9 in a concentration of 0.2µM. The concentration of the specific oncogenic HPV primers were unpaired by a factor of 100, in order to perform an asymmetrical amplification in which an excess was generated of one of the bands (the band primed by primer A9) in a simple band structure that can later be used as a hybridisation probe. In addition, 0.3 microlitres of dUTP marked with digoxygenin was added to each phial.

The appropriate volume of stabilising mixture was added to each phial as shown in Table 1. The tubes thus prepared were desiccated in a centrifugal evaporator Eppendorf model 5301 at temperatures ranging from 10°C to 60°C for times ranging from 30 to 120 minutes. These temperatures and times will depend on the final volume of mixture to be desiccated.

After desiccation, t10 picograms of pOncl plasmid were added to each tube and the volume was adjusted to 50 microlitres with sterile distilled H₂O. The amplification cycles were then performed, consisting of an initial incubation at 95°C for 10 followed by 35 cycles of denaturation (95°C, 1 minute), annealing (55°C, 1 minute) and extension (72°C, 1 minute), using a MiniCycler (MJ Research) thermocycler.

To each of the amplified tubes was added the suitable volume of each of the stabilising mixtures shown in Table 1. The tubes thus prepared were centrifuged for 10 seconds at 4000 rpm in a tabletop centrifuge (Eppendorf) to ensure that the mixture remained at the bottom of the capillary tube. Finally they were desiccated in a centrifugal evaporator Eppendorf model 5301 at temperatures ranging from 10°C to 60°C for times ranging from 30 to 120 minutes.

After desiccation, the tubes were conserved at the temperatures and for the times shown in Table 1. After the times shown in Table 1 had elapsed, their activity was tested using them as probes for detection of specific HPV 16 sequences by molecular hybridisation. To do so the tubes were resuspended in 50 microlitres of sterile distilled H₂O and added to an ELISA plate top which the A11 nucleotide (specific for HPV 16 and with the opposite polarity to the asymmetric band obtained in the amplification reaction) had been previously fixed. The plate was then incubated for 30 minutes at 45°C and then revealed after incubation with the anti-DIG-AP conjugate, according to the hybridisation protocol instructions recommended for the DNA-Bind plates.

The results were positive in all cases. This experiment showed the possibility of performing reactions of incorporation of nucleotides marked with digoxygenin using desiccated mixtures including said nucleotides. It also confirmed the possibility of stabilising the amplified products marked with digoxygenin, and their later use as molecular hybridisation probes (Table 1).

### EXAMPLE 10

### Stabilisation of nucleic acids and proteins immobilised in different substrates

### 10.1 Stabilisation of nucleic acids immobilised in ELISA microplates

To test the possibility of stabilising nucleic acids that have been previously immobilised in ELISA microplates for use in subsequent colorimetric or fluorescent assays, NucleoLink (Costar) plates were used especially designed to immobilise biomolecules in a specially treated plastic, which maintains a high degree of transparency that allows its use in colorimetry assays, while also withstanding high temperatures. These characteristics make it appropriate for use in gene amplification reactions and subsequent colorimetric assays.

To the plate dimples was fixed the primer A13 (^{5'}-P-GAGCTGTCGCTTAATTGCTC-^{3'}) complementary to the HPV genome, phosphorylated at end 5' (P in the sequence) to allow fixing to the dimples. The probe was fixed by the protocol recommended by the distributor of the NucleoLink plates. After fixing the probe, a mixture was added to each dimple comprising 1 microlitre of DNA polymerase (1 U/µl) conserved in its storage buffer, 5 microlitres of the reaction buffer, 1 microlitre of a solution containing equimolar proportions of the four deoxyribonucleotides (dNTP's) participating in the DNA amplification reaction (dATP, dCTP, dGTP and dTTP) and 1µl of the primer A9 in a concentration of 20µM. Finally, the appropriate volume of each of the stabilising mixtures shown in Table 1 was added to each dimple.

The plates thus prepared were desiccated in a Memmert vacuum oven (model V0400) at 25°C and 50 mbar pressure for 1 h. 30 min. After desiccation, the tubes were conserved at the temperatures and for the times shown in Table 1. At the end of the times shown in Table 1 their functionality was tested by performing an amplification reaction. To do so, 50 microlitres of a dilution of 0.5 pmol/microlitre of pOnc plasmid was added to each dimple. The amplification reaction was performed with the following cycles: an initial incubation at 95°C for 10 minutes, followed by 35 cycles of denaturation (95°C, 1 minute), annealing (55°C, 1 minute) and extension (72°C, 1 minute) using a Minicycler (MJ Research) thermocycler.

After the amplification reaction, the colorimetric detection was performed of the band amplified and fixed in the solid substrate, following the protocol recommended by the manufacturer of the NucleoLink plates. The oligonucleotide used as a hybridisation probe was A11, marked with biotin. The conjugate used was streptoavidin-HRP and the substrate was ABTS.

The analysis of the results showed that the plate retained their amplification capacity, indicating the correct stabilisation of the immobilised probe. Likewise, it was confirmed that including the stabilisation mixture did not interfere with subsequent colorimetric assays (Table 1).

### 10.2 Stabilisation of proteins immobilised in ELISA microplates

To test the possibility of stabilising proteins that have been previously immobilised in ELISA microplates for use in subsequent colorimetric or fluorescent assays, as in Example 10.1 NucleoLink (Costar) plates were used. To do so each dimple was incubated with 1µg of antidigoxygenin antibody (Roche). After incubation for 30 minutes at 37°C, the appropriate volume of each of the stabilising mixtures shown in Table 1 was added to each dimple.

The plates thus prepared were desiccated in a Memmert vacuum oven at 25°C and 50 mbar pressure for 1 h. 30 min. After desiccation, the tubes were conserved at the temperatures and for the times shown in Table 1. At the end of the times shown in Table 1 the stability of the immobilised antibody was tested by a colorimetry assay.

An amplification product of the HPV genome, amplified with the pair of primers A12 (marked with digoxygenin at end 5') and A11 (marked with biotin at end 5'). The amplified product was incubated for 30 minutes in the dimples with the antibody immobilised. The amplified products immobilised in the plate by the union of the digoxygenin group of the amplicons and the immobilised anti-digoxygenin group were analysed by a colorimetric assay. To do so, each dimple was incubated with streptoavidin conjugated with HRP and revealed after washing with ABTS. The colorimetric reaction was finally analysed by measuring absorption at 415 nm.

The analysis of the results showed that the antibody immobilised in the plate retained its capacity to recognise and bind the digoxygenin group, indicating the correct stabilisation of the immobilised protein. Likewise, it was confirmed that including the stabilisation mixture did not interfere with subsequent colorimetric assays (Table 1).

### 10.3 Immobilisation of nucleic acids immobilised in modified glasses

To test the possibility of stabilising nucleic acids that have been previously immobilised in pre-treated glass supports, the usual format of microarray systems, two different alternatives were tested.

Approach 1. Stabilisation of supports with previously fixed nucleic acids. HPV specific probes were fixed to glass supports pre-treated with polylysiun, aldehyde, epoxy, superepoxy and silane. To do so, in each support slide 1 microlitre spots were applied of the solution of each oligonucleotide at a concentration of 20 µM in the conditions indicated for each glass format. The slides with the immobilised oligonucleotides were then coated with a thin layer of each of the stabilising mixtures and then desiccated in a vacuum oven at 25°C and 50 mbar pressure. Finally, the slides coated with the desiccated thin film were stored in the conditions and for the times shown in Table 1.

Approach 2. Fixation to the slides of the nucleic acids pre-mixed with the stabilising mixtures. A mixture was prepared of each of the probes with the suitable volumes of the stabilising mixtures described in Table 1. Then the mixtures of oligonucleotides were applied to the various slides with the fixing conditions indicated for each glass format. Finally, the slides were vacuum dried in the conditions and for the times indicated in Table 1.

To test the viability of each slide prepared, a colorimetric assay was performed with formation of precipitate products. To do so, the slides were washed for 5 minutes in PBS 1X at room temperature to eliminate the protective layer. Then the slides were incubated in the presence of an amplified and previously denatured HPV 16 product using the probes A11 and A12. The slides were then incubated with the conjugate streptoavidin HRP and revealed with DAB.

The analysis of the results showed that the oligonucleotides immobilised in the slides, regardless of their format and both when using the approach of stabilising the entire slide or stabilising the oligonucleotides prior to application, retained their capacity to recognise and bind complementary sequences of nucleic acids. It was also confirmed that the inclusion of the stabilisation mixture did not interfere in subsequent colorimetric assays (Table 1).

### 10.4 Immobilisation of proteins in modified glasses

To test the possibility of stabilising proteins that have been previously immobilised in pre-treated glass supports, the usual format of microarray systems, two different alternatives were tested as in the previous experiment.

Approach 1. Stabilisation of supports with previously fixed protein. Anti-digoxygenin antibodies were fixed to glass supports pre-treated with aldehyde. To do so, in each support slide 1 microlitre spots were applied of the solution of each protein at a concentration of 30 ng/µl. The slides with the immobilised protein were then coated with a thin layer of each of the stabilising mixtures and then desiccated in a vacuum oven at 25°C and 50 mbar pressure. Finally, the slides coated with the desiccated thin film were stored in the conditions and for the times shown in Table 1.

Approach 2. Fixation to the slides of the protein pre-mixed with the stabilising mixtures. A mixture was prepared of each of the probes with the suitable volumes of the stabilising mixtures described in Table 1. Then the mixtures of protein were applied to the various slides with the fixing conditions indicated for each glass format. Finally, the slides were vacuum dried in the conditions and for the times indicated in Table 1.

To test the viability of each slide prepared, a colorimetric assay was performed with formation of precipitate products. To do so, the slides were washed for 5 minutes in PBS 1X at room temperature to eliminate the protective layer. Then the slides were incubated in the presence of an amplified and non-denatured product of *P. falciparum* using the probes A11 (marked with biotin at end 5') and A12 (marked with digoxygenin at end 5'). The slides were then incubated with the conjugate streptoavidin-HRP and revealed with DAB.

The analysis of the results showed that the antidigoxygenin antibodies immobilised in the slides, both when using the approach of stabilising the entire slide or stabilising the protein prior to application, retained their capacity to recognise and bind the digoxygenin group. It was also confirmed that the inclusion of the stabilisation mixture did not interfere in subsequent colorimetric assays (Table 1).

**Table 1**

| | | | | | | | | | | | | | | |
|---|---|---|---|---|---|---|---|---|---|---|---|---|---|---|
| **Results of the activity of stabilising mixtures in the various examples tested** | | | | | | | | | | | | | | |
| The results are expressed in the following way: Ng, negative results; +, weak positive, under 50% of the activity of the fresh mixture; ++, positive with 50-90% of the activity of the fresh mixture; +++, positive with activity level not less than 90% of the activity of the fresh mixture. PVP: polyvinylpyrrolidin. PEG: polyethyleneglycol. G. Arabic: Gum Arabic. | | | | | | | | | | | | | | |

| MIXTURE | EXAMPLES | | | | | | | | | | | | | |
|---|---|---|---|---|---|---|---|---|---|---|---|---|---|---|
| | T (°C) | 1 | 2 | 3 | 4 | 5 | 6 | 7 | 8 | 9 | 10.1 | 10.2 | 10.3 | 10.4 |
| Sucrose | 4 | + | + | + | + | + | + | + | + | + | + | + | + | + |
| | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Rafinose | 4 | + | + | + | + | + | + | + | + | + | + | + | + | + |
| | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Palatinitol | 4 | + | + | + | + | + | + | + | + | + | + | + | + | + |
| | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Melezitose | 4 | + | + | + | + | + | + | + | + | + | + | + | + | + |
| | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Glycerol | 4 | ++ | ++ | ++ | ++ | ++ | ++ | ++ | ++ | ++ | ++ | ++ | ++ | ++ |
| | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Lysine | 4 | + | + | + | + | + | + | + | + | + | + | + | + | + |
| | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Glucogen | 4 | ++ | ++ | ++ | ++ | ++ | ++ | ++ | ++ | ++ | ++ | ++ | ++ | ++ |
| | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Trehalose | 4 | ++ | ++ | ++ | ++ | ++ | ++ | ++ | ++ | ++ | ++ | ++ | ++ | ++ |
| | 2 5 | + | + | + | + | + | + | + | + | + | + | + | + | + |
| | 4 5 | Ng | Ng | Ng | Ng | Ng | Ng | Ng | Ng | Ng | Ng | Ng | Ng | Ng |
| Sorbitol | 4 | ++ | ++ | ++ | ++ | ++ | ++ | ++ | ++ | ++ | ++ | ++ | ++ | ++ |
| | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Trehalose + Lysine | 4 | ++ | ++ | ++ | ++ | ++ | ++ | ++ | ++ | ++ | ++ | ++ | ++ | ++ |
| | 2 5 | + | + | + | + | + | + | + | + | + | + | + | + | + |
| | 4 5 | Ng | Ng | Ng | Ng | Ng | Ng | Ng | Ng | Ng | Ng | Ng | Ng | Ng |
| Trehalose + PVP | 4 | + | + | + | + | + | + | + | + | + | + | + | + | + |
| | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Sorbitol + | 4 | + | + | + | + | + | + | + | + | + | + | + | + | + |
| PEG | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Rafinose + Betaine | 4 | + | + | + | + | + | + | + | + | + | + | + | + | + |
| | 2 5 | + | + | + | + | + | + | + | + | + | + | + | + | + |
| | 4 5 | Ng | Ng | Ng | Ng | Ng | Ng | Ng | Ng | Ng | Ng | Ng | Ng | Ng |
| Melezitose + lysine | 4 | + | + | + | + | + | + | + | + | + | + | + | + | + |
| | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Melezitose + glucogen | 4 | + | + | + | + | + | + | + | + | + | + | + | + | + |
| | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Lysine + glucogen | 4 | + | + | + | + | + | + | + | + | + | + | + | + | + |
| | 2 5 | + | + | + | + | + | + | + | + | + | + | + | + | + |
| | 4 5 | Ng | Ng | Ng | Ng | Ng | Ng | Ng | Ng | Ng | Ng | Ng | Ng | Ng |
| Melezitose + lysine + PEG | 4 | ++ | ++ | ++ | ++ | ++ | ++ | ++ | ++ | ++ | ++ | ++ | ++ | ++ |
| | 2 5 | + | + | + | + | + | + | + | + | + | + | + | + | + |
| | 4 5 | Ng | Ng | Ng | Ng | Ng | Ng | Ng | Ng | Ng | Ng | Ng | Ng | Ng |
| Melezitose + lysine + PVP | 4 | + | + | + | + | + | + | + | + | + | + | + | + | + |
| | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Melezitose + lysine + dextrane | 4 | Ng | Ng | Ng | Ng | Ng | Ng | Ng | Ng | Ng | Ng | Ng | Ng | Ng |
| | 2 5 | Ng | Ng | Ng | Ng | Ng | Ng | Ng | Ng | Ng | Ng | Ng | Ng | Ng |
| | 4 5 | Ng | Ng | Ng | Ng | Ng | Ng | Ng | Ng | Ng | Ng | Ng | Ng | Ng |
| Melezitose + | 4 | +++ | +++ | +++ | +++ | +++ | +++ | +++ | +++ | +++ | +++ | +++ | +++ | +++ |
| lysine + glucogen | 2 5 | ++ | ++ | ++ | ++ | ++ | ++ | ++ | ++ | ++ | ++ | ++ | ++ | ++ |
| | 4 5 | + | + | + | + | + | + | + | + | + | + | + | + | + |
| Melezitose + lysine + G. Arabic | 4 | +++ | +++ | +++ | +++ | +++ | +++ | +++ | +++ | +++ | +++ | +++ | +++ | +++ |
| | 2 5 | ++ | ++ | ++ | ++ | ++ | ++ | ++ | ++ | ++ | ++ | ++ | ++ | ++ |
| | 4 5 | + | + | + | + | + | + | + | + | + | + | + | + | + |
| Melezitose + betaine + glucogen | 4 | + | + | + | + | + | + | + | + | + | + | + | + | + |
| | 2 5 | + | + | + | + | + | + | + | + | + | + | + | + | + |
| | 4 5 | Ng | Ng | Ng | Ng | Ng | Ng | Ng | Ng | Ng | Ng | Ng | Ng | Ng |
| Rafinose + betaine + glucogen | 4 | + | + | + | + | + | + | + | + | + | + | + | + | + |
| | 2 5 | + | + | + | + | + | + | + | + | + | + | + | + | + |
| | 4 5 | Ng | Ng | Ng | Ng | Ng | Ng | Ng | Ng | Ng | Ng | Ng | Ng | Ng |
| Melezitose + glucogen + trehalose + lysine | 4 | +++ | +++ | +++ | +++ | +++ | +++ | +++ | +++ | +++ | +++ | +++ | +++ | +++ |
| | 2 5 | ++ | ++ | ++ | ++ | ++ | ++ | ++ | ++ | ++ | ++ | ++ | ++ | ++ |
| | 4 5 | ++ | ++ | ++ | ++ | ++ | ++ | ++ | ++ | ++ | ++ | ++ | ++ | ++ |
| Melezitose +G.Arabic+ trehalose + lysine | 4 | +++ | +++ | +++ | +++ | +++ | +++ | +++ | +++ | +++ | +++ | +++ | +++ | +++ |
| | 2 5 | ++ | ++ | ++ | ++ | ++ | ++ | ++ | ++ | ++ | ++ | ++ | ++ | ++ |
| | 4 5 | ++ | ++ | ++ | ++ | ++ | ++ | ++ | ++ | ++ | ++ | ++ | ++ | ++ |
| Palatinol+ glucogen+ trehalose+ lysine | 4 | +++ | +++ | +++ | +++ | +++ | +++ | +++ | +++ | +++ | +++ | +++ | +++ | +++ |
| | 2 5 | ++ | ++ | ++ | ++ | ++ | ++ | ++ | ++ | ++ | ++ | ++ | ++ | ++ |
| | 4 5 | ++ | ++ | ++ | ++ | ++ | ++ | ++ | ++ | ++ | ++ | ++ | ++ | ++ |
| Palatinol+ G.Arabic+ trehalose+ lysine | 4 | +++ | +++ | +++ | +++ | +++ | +++ | +++ | +++ | +++ | +++ | +++ | +++ | +++ |
| | 2 5 | ++ | ++ | ++ | ++ | ++ | ++ | ++ | ++ | ++ | ++ | ++ | ++ | ++ |
| | 4 5 | ++ | ++ | ++ | ++ | ++ | ++ | ++ | ++ | ++ | ++ | ++ | ++ | ++ |
| Rafinose+ | 4 | +++ | +++ | +++ | +++ | +++ | +++ | +++ | +++ | +++ | +++ | +++ | +++ | +++ |
| glucogen+ betaine | 2 5 | ++ | ++ | ++ | ++ | ++ | ++ | ++ | ++ | ++ | ++ | ++ | ++ | ++ |
| | 4 5 | ++ | ++ | ++ | ++ | ++ | ++ | ++ | ++ | ++ | ++ | ++ | ++ | ++ |

## Claims

1. A stabilised composition that contains:
(i) a component (A) selected from the group formed by:
- a compound comprising a fluorophore (component A1);
- a compound comprising a first member of a specific binding pair able to recognise and interact with a second member of said specific binding pair (component A2);
- an enzymatic activity that catalyses a colorimetric or chemoluminescent reaction (component A3);
- a conjugate comprising an enzymatic activity that catalyses a colorimetric or chemoluminescent reaction and a member of a specific binding pair able to recognise and bind a second member of said specific binding pair component (A4);
- one or more compounds joined to a solid support (component A5);
- their mixtures; and
(ii) a component (B) constituted by a stabilising mixture that comprises:
- at least one protective agent against desiccation (component B1);
- at least one inhibitor of the condensation reaction between carbonyl or carboxyl groups and amine or phosphate groups (component B2); and
- at least one inert polymer able to generate a grid structure that prevents the mobility of the desiccated reagents (component B3).

2. Composition according to claim 1, that also comprises a component selected from among water, enzymes, reagents for the reactions in which said enzymes participate and mixtures thereof.

3. Composition according to any of claims 1 or 2 comprising, in addition to components (A) and (B), an enzyme that catalyses the polymerisation of nucleic acids, together with all or some of the components needed to carry out said nucleic acid polymerisation reaction.

4. Composition according to any of the previous claims in which the humidity level is equal to or under 30%, preferably between 1 % and 20%.

5. Composition according to claim 1 that comprises a component A1, A2, A3 or A4.

6. Composition according to claim 1 in which said component A1 comprises a fluorescent compound; a nucleotide marked with a fluorophore; an oligonucleotide containing a fluorophore and, optionally, a compound that modulates the fluorescence (a quencher); a polynucleotide containing a fluorophore and, optionally, a compound that modulates the fluorescence (a quencher); a peptide marked with a fluorophore; a protein marked with a fluorophore; and antigen marked with a fluorophore; or an antibody marked with a fluorophore.

7. Composition according to claim 1 in which said component A2 is selected from among a compound comprising biotin, phycoerythrin, digoxygenin, an antibody, a peptide, a protein, a nucleic acid or a compound that can be fixed by adsorption or by covalent binding.

8. Composition according to claim 7 in which said component A2 is selected from among an antibody joined to biotin or phycoerythrin, an antigen joined to biotin or phycoerythrin, an oligonucleotide joined to digoxygenin and a polynucleotide joined to digoxygenin.

9. Composition according to claim 1 in which said component A3 comprises an enzymatic activity selected from among peroxidase and alkaline phosphatase activities.

10. Composition according to claim 1 in which said component A3 comprises an enzymatic activity selected from among β-D-galactose, xanthene oxidase, glucose oxidase, luciferase, green fluorescent protein (GFP), peroxidase and alkaline phosphatase.

11. Composition according to claim 1 in which said component A4 comprises a conjugate avidin - enzymatic activity able to catalyse a colorimetric or chemoluminescent reaction, a conjugate streptavidin - enzymatic activity able to catalyse a colorimetric or chemoluminescent reaction, or a conjugate antidigoxygenin - enzymatic activity able to catalyse a colorimetric or chemoluminescent reaction.

12. Composition according to claim 1, in which said component A5 comprises a solid support containing one or more compounds immobilised on said solid support, which can be used as a component of fluorimetric, colorimetric, chemoluminescent detection systems, electrical conductivity systems or index of refraction change systems.

13. Composition according to claim 12, in which said solid support is selected from among solid supports of plastic, glass and gold surfaces, optionally treated to allow adsorption or covalent bonding of macromolecules.

14. Composition according to claim 12, in which said compounds or compounds immobilised on said solid support comprise biomolecules.

15. Composition according to claim 14, in which said biomolecules are selected from among nucleic acids and proteins.

16. Composition according to claim 12, in which said component A5 is selected from among ELISA dimple microplates, chips and microarrays containing, immobilised on their surfaces, one or more biomolecules selected from among nucleic acids and proteins.

17. Composition according to claim 1, in which said component B1 comprises at least one non-reducing carbohydrate.

18. Composition according to claim 17 in which said non-reducing carbohydrate is selected from the group formed by a non-reducing disaccharide, a non-reducing trisaccharide and their mixtures.

19. Composition according to claim 17, in which said non-reducing carbohydrate is selected from among palatinol, trehalose, rafinose, melezitose and their mixtures.

20. Composition according to any of claims 17 to 19, which also comprises a polyol.

21. Composition according to claim 20, in which said polyol is selected from among glycerol, sorbitol and their mixtures

22. Composition according to claim 1, in which said component B2 is selected from among an amino acid, betaine, aminoguanidine, derivatives of aminoguanidine and their mixtures.

23. Composition according to claim 22 in which said amino acid is selected among lysine, arginine, tryptophane and their mixtures.

24. Composition according to claim 1 in which the component B3 is selected from among the group formed by polyvinyl pyrrolidone (PVP), polyethylene glycol (PEG) in various degrees of polymerisation, dextrane, starch, Ficoll, glucogen, gum Arabic and their mixtures.

25. Composition according to claim 3, which comprises an intercalated fluorophore between dsDNA's or a compound comprising a fluorophore and, optionally, a quencher, selecting said compound from among a nucleotide, an oligonucleotide and a polynucleotide, and also an enzyme that catalyses the polymerisation of said nucleic acids.

26. Composition according to claim 1, which comprises an antibody conjugated to a fluorescent compound, or an antibody conjugated to biotin, or a conjugated antibody that comprises an enzymatic activity that catalyses a colorimetric or chemoluminescent reaction and avidin or streptavidin.

27. Composition according to claim 1, which comprises an antigen conjugated to a fluorescent compound, or an antigen conjugated to biotin, or a conjugate that comprises an enzymatic activity that catalyses a colorimetric or chemoluminescent reaction and avidin or streptavidin.

28. Kit comprising at least one container holding a composition according to any of claims 1 to 27.

29. Kit according to claim 28, selected among:
a kit for real-time amplification and/or detection of specific sequences of nucleic acids that comprises, at least, one container holding a composition that comprises an enzyme that catalyses the polymerisation of nucleic acids and a component A1 selected from among: (i) dATP, dGTP, dCTP and dTTP or dUTP marked with fluorophores; (ii) a primer marked with a fluorophore; (iii) a probe containing a fluorophore and, optionally a quencher; and (iv) a fluorescent compound intercalated in dsDNA's;
a kit for nucleic acid sequencing by gene amplification that comprises at least one container holding a composition that comprises an enzyme that catalyses the polymerisation of nucleic acids and a component A1 selected from among: (i) dATP, dGTP, dCTP and dTTP or dUTP marked with fluorophores; and (ii) a primer marked with a fluorophore;
a kit for nucleic acid sequencing by nucleic acid elongation reactions that comprises at least one container holding a composition that comprises an enzyme that catalyses the polymerisation of nucleic acids and a component A1 selected from among: (i) dATP, dGTP, dCTP and dTTP marked with fluorophores; and (ii) a primer marked with a fluorophore;
a kit for detection of nucleic acids comprising, at least, one container holding a composition according to claim 1, said composition comprising a compound that includes a member of a specific binding pair conjugated to an enzymatic activity that catalyses a colorimetric or chemoluminescent reaction;
a kit for detection of antibodies comprising, at least, one container holding a composition according to claim 1, said composition comprising a compound that includes a member of a specific binding pair conjugated to an enzymatic activity that catalyses a colorimetric or chemoluminescent reaction; and
a kit for detection of antigens comprising, at least, one container holding a composition according to claim 1, said composition comprising a compound that includes a member of a specific binding pair conjugated to an enzymatic activity that catalyses a colorimetric or chemoluminescent reaction.

30. A procedure for preparing a stabilised composition with a humidity level equal to or less than 30% that involves:
a) bringing together in a single container:
an aqueous solution comprising at least one component (A) selected from among the group formed by:
- a compound comprising a fluorophore (component A1);a compound comprising a first member of a specific binding pair able to recognise and interact with said specific binding pair (component A2);
- an enzymatic activity that catalyses a colorimetric or chemoluminescent reaction (component A3);
- a conjugate comprising an enzymatic activity that catalyses a colorimetric or chemoluminescent reaction and a member of a specific binding pair able to recognise and bind a second member of said specific binding pair component (A4); and
- their mixtures; and
an aqueous solution comprising at least one component (B) constituted by:
- at least one protective agent against desiccation (component B1);
- at least one inhibitor of the condensation reaction between carbonyl or carboxyl groups and amine or phosphate groups (component B2); and
- at least one inert polymer able to generate a grid structure that prevents the mobility of the desiccated reagents (component B3)
to obtain an aqueous solution that comprises said components A and B; and
b) removing all or part of the water contained in said aqueous solution containing the components A and B obtained in stage a) until obtaining a composition that comprises said components A and B and has a humidity level equal to or less than 30%.

31. Procedure according to claim 30, in which the removal of all or part of the water present in the aqueous solution resulting from mixing the aqueous solutions comprising components A and B in the container is performed by lyophilisation, fluidised bed desiccation, desiccation at room temperature and atmospheric pressure, desiccation at room temperature and reduced pressure, desiccation at high temperature and atmospheric pressure or desiccation at high temperature and reduced pressure.

32. Procedure according to claim 31 in which the removal of all or part of the water contained in the aqueous solution resulting from mixing the aqueous solutions comprising components A and B in the container is performed by desiccation at a temperature between 15°C and 60°C and at a reduced pressure lower than atmospheric pressure.

33. A procedure for preparing a stabilised composition comprising a solid support with one or more immobilised and stabilised components, with a humidity level equal to or under 30%, that involves:
bringing together a component (A) that consists of a solid support containing one or more products previously immobilised on said solid support with a component (B) that consists of a stabilising mixture that comprises:
- at least one protective agent against desiccation (component B1);
- at least one inhibitor of the condensation reaction between carbonyl or carboxyl groups and amine or phosphate groups (component B2); and
- at least one inert polymer able to generate a grid structure that prevents the mobility of the desiccated reagents (component B3); and
removing all or part of the water contained in the resulting mixture of said components (A) and (B) until obtaining a composition that comprises a solid support with one or more immobilised and stabilised compounds and said component (B) and with a humidity level equal to or under 30%.

34. Procedure according to claim 33, in which said component (A) comprises a solid support made of plastic, glass or gold surfaces, optionally pre-treated to allow the adsorption or covalent bonding of compounds.

35. Procedure according to any of claims 33 or 34 in which said compound or compounds immobilised in the solid support comprises one or more biomolecules.

36. Procedure according to claim 35 in which said biomolecule is selected from among nucleic acids, proteins, peptides and antibodies.

37. A procedure for preparing a stabilised composition that comprises a solid support containing one or more immobilised and stabilised components, with humidity level equal to or under 30%, that involves:
- bringing together (i) a solid support, optionally treated to immobilise compounds, with (ii) one or more compounds stabilised by mixing said compound or compounds with a component (B) that consists of a stabilising mixture that comprises:
- at least one protective agent against desiccation (component B1);
- at least one inhibitor of the condensation reaction between carbonyl or carboxyl groups and amine or phosphate groups (component B2); and
- at least one inert polymer able to generate a grid structure that prevents the mobility of the desiccated reagents (component B3); and
removing all or part of the water contained in the resulting mixture until obtaining a composition that comprises a solid support with one or more immobilised and stabilised compounds and said component (B), with a humidity level equal to or under 30%.

38. Procedure according to claim 37, in which said solid support is a solid support made of plastic, glass or gold surfaces, optionally pre-treated to allow the adsorption or covalent bonding of compounds.

39. Procedure according to any of claims 37 or 38, in which said stabilised compound or compounds comprises one or more biomolecules.

40. Procedure according to claim 39 in which said biomolecule is selected from among nucleic acids, proteins, peptide and antibodies.

41. A kit containing a stabilised composition comprising a solid support that contains one or more immobilised and stabilised compounds, with a humidity level equal to or less than 30%, that can be obtained by the procedure of any of claims 33 to 40, as a detection substrate in colorimetric, fluorescent or chemoluminescent systems.

42. A kit containing a stabilised composition comprising a solid support that contains one or more immobilised and stabilised compounds, with a humidity level equal to or less than 30%, that can be obtained by the procedure of any of claims 33 to 40, as a detection substrate in a method of analysing results such as a measure of electrical conductivity or index of refraction.

43. Use of a composition according to any of claims 1 to 27, or of a kit according to any of claims 28, 29, 41 or 42 to perform a fluorimetric, colorimetric or chemoluminescent assay.
